# EUROPEAN PATENT APPLICATION

(11) **EP 4 083 202 A1**
(43) Date of publication of application: **02.11.2022**
(21) Application number: 20908327.8
(22) Date of filing: 25.12.2020
(51) Int. Cl.: C12N 15/09, C12Q 1/6844, C12Q 1/6876

(54) **PRIMER SET AND METHOD FOR DETECTING TARGET NUCLEIC ACID USING SAME**

(30) Priority: 27.12.2019 JP 2019239045
(71) Applicant: Kaneka Corporation, Osaka-shi, Osaka 530-8288 (JP)
(72) Inventor: MIYAMOTO Shigehiko, Takasago-shi, Hyogo 676-8688 (JP)
(74) Representative: Vossius & Partner Patentanwälte Rechtsanwälte mbB
(86) International application number: PCT/JP2020/048826
(87) International publication number: WO 2021/132596

(57) **Abstract**

One or more embodiments of the present invention are intended to dissolve the problem of lowering in reaction efficiency of the nucleic acid amplification reaction using a primer with a polynucleotide tag to prepare an amplified product of a target nucleic acid that can be detected on a solid-phase support. One or more other embodiments of the present invention relate to a set of primers comprising: the first primer comprising the first polynucleotide comprising, at the 3' terminus, polynucleotide A capable of hybridizing to a complementary strand of partial polynucleotide A' at the 5' terminus of the target nucleic acid and the first polynucleotide tag, which is a polynucleotide independent of the nucleic acid amplification reaction; the second primer comprising the second polynucleotide comprising, at the 3' terminus, polynucleotide B capable of hybridizing to partial polynucleotide B' at the 3' terminus of the target nucleic acid; and the third primer comprising the third polynucleotide hybridizing to the complementary strand of the target nucleic acid competitively with the polynucleotide A of the first primer but comprising no polynucleotide independent of the nucleic acid amplification reaction.

## Description

### Technical Field

One or more embodiments of the present invention relate to a set of primers for preparing an amplified product of a target nucleic acid that can be detected on a solid-phase support via a nucleic acid amplification reaction.

One or more embodiments of the present invention also relate to a kit for detecting a target nucleic acid comprising the set of primers.

One or more embodiments of the present invention also relate to a method for detecting a target nucleic acid comprising performing a nucleic acid amplification reaction using the set of primers.

One or more embodiments of the present invention also relate to a multiplex set of primers comprising 2 or more sets of primers described above.

### Background Art

In recent years, a chromatography-based gene detection method has drawn attention as a rapid and simple method for detecting a target nucleic acid with excellent operability (e.g., Patent Document 1). This method is summarized as follows. A target nucleic acid is amplified via a nucleic acid amplification reaction using a polynucleotide primer with a polynucleotide tag independent of the nucleic acid amplification reaction to generate a target nucleic acid fragment with a polynucleotide tag. On the other hand, a probe to bind to the polynucleotide tag (e.g., a polynucleotide complementary to the polynucleotide tag described above) is immobilized in advance to a chromatography test strip. A sample containing the target nucleic acid fragment with the tag is then applied to the test strip, the sample is allowed to spread in the test strip by capillary action, and the target nucleic acid fragment is captured and detected by the probe.

Meanwhile, an isothermal nucleic acid amplification method in which a gene of interest is amplified under isothermal conditions, unlike PCR that requires a thermal cycler to change sample temperatures, has been developed as a nucleic acid amplification method. An isothermal nucleic acid amplification method is suitable for POC (point of care) testing because, for example, it can be performed with a simple apparatus, a reaction period is short, and detection sensitivity is high. Examples of known isothermal nucleic acid amplification methods include the RPA (recombinase polymerase amplification) method, the TRIAmp (tandem repeat-mediated isothermal amplification) method, the LAMP (loop-mediated isothermal amplification) method, and the HAD (helicase-dependent amplification) method.

Examples of prior art documents that disclose a use of a polynucleotide primer with a polynucleotide tag in an isothermal nucleic acid amplification method, include Patent Document 2 and Patent Document 3 indicated below.

Patent Document 2 discloses that tag sequences and label-binding substances are linked to given 2 primers among 6 primers used in the LAMP method and that an amplified product obtained using the primers is captured and detected on a solid-phase support.

Patent Document 3 discloses an isothermal nucleic acid amplification method based on a novel principle comprising adding a tag independent of the nucleic acid amplification reaction to a primer.

### Prior Art Documents

### Patent Documents

Patent Document 1: JP 2016-73312 A
Patent Document 2: WO 2017/43114
Patent Document 3: WO 2018/038232
Patent Document 4: JP Patent No. 5688702

### Non-Patent Documents

Non-Patent Document 1: Frontiers in Microbiology, vol. 8, Article 192

### Summary of the Invention

### Objects to Be Attained by the Invention

The present inventors found that: when using primers with polynucleotide tags in a nucleic acid amplification reaction, such as PCR and an isothermal nucleic acid amplification, nucleic acid amplification reaction efficiency may be lowered, compared with a case using primers without tags. In addition, the present inventor found that: in an isothermal amplification reaction using strand displacing polymerase, nucleic acid amplification reaction efficiency may be lowered to a significant extent by using primers with polynucleotide tags.

Thus, one or more embodiments of the present invention are intended to dissolve the problem of reduction in reaction efficiency of a nucleic acid amplification reaction using primers with polynucleotide tags for preparing an amplified product of a target nucleic acid that can be detected on a solid-phase support.

### Means for Attaining the Objects

The present descriotion discloses the inventions described below as means for attaining the above objects.
(1) A set of primers for preparing an amplified product of a target nucleic acid that can be detected on a solid-phase support via a nucleic acid amplification reaction,
   wherein the set of primers comprises a first primer, a second primer, and a third primer,
   wherein the first primer comprises:
      a first polynucleotide comprising, at the 3' terminus, polynucleotide A capable of hybridizing to a complementary strand of partial polynucleotide A' at the 5' terminus of a target nucleic acid; and
      a first polynucleotide tag, which is a polynucleotide independent of the nucleic acid amplification reaction, linked to the 5' terminus of the first polynucleotide,
      wherein the second primer comprises a second polynucleotide comprising, at the 3' terminus, polynucleotide B capable of hybridizing to partial polynucleotide B' at the 3' terminus of the target nucleic acid, and
      wherein the third primer comprises a third polynucleotide comprising, at the 3' terminus, polynucleotide C capable of hybridizing to the complementary strand of the target nucleic acid competitively with the polynucleotide A of the first primer, and comprises no polynucleotide independent of the nucleic acid amplification reaction.
(2) The set of primers according to (1), wherein the polynucleotide C of the third primer is capable of hybridizing to a polynucleotide having 50% or more nucleotides within a complementary strand of the partial polynucleotide A' of the target nucleic acid.
(3) The set of primers according to (1) or (2), wherein the proportion of the third primer to the total amount of the first primer and the third primer is 1 mol% to 90 mol%.
(4) The set of primers according to (3), wherein the proportion of the third primer to the total amount of the first primer and the third primer is 2.5 mol% to 75 mol%.
(5) The set of primers according to any one of (1) to (4), wherein, in the first primer, the 3' terminus of the first polynucleotide tag is linked to the 5' terminus of the first polynucleotide via an inhibitor that inhibits the nucleic acid amplification reaction.
(6) The set of primers according to any one of (1) to (5), wherein the second primer further comprises a label substance linked to the second polynucleotide.
(7) The set of primers according to (6), wherein the label substance is biotin, a fluorescent dye, or hapten.
(8) The set of primers according to any one of (1) to (5),
   wherein the set of primers further comprises a fourth primer,
   wherein the second primer further comprises a second polynucleotide tag, which is a polynucleotide independent of the nucleic acid amplification reaction, linked to the 5' terminus of the second polynucleotide, and
   wherein the fourth primer comprises a fourth polynucleotide comprising, at the 3' terminus, polynucleotide D capable of hybridizing to the target nucleic acid competitively with the polynucleotide B of the second primer, and comprises no polynucleotide independent of the nucleic acid amplification reaction.
(9) The set of primers according to (8), wherein the polynucleotide D of the fourth primer is capable of hybridizing to a polynucleotide having 50% or more nucleotides within the partial polynucleotide B' of the target nucleic acid.
(10) The set of primers according to (8) or (9), wherein the proportion of the fourth primer to the total amount of the second primer and the fourth primer is 1 mol% to 90 mol%.
(11) The set of primers according to (10), wherein the proportion of the fourth primer to the total amount of the second primer and the fourth primer is 2.5 mol% to 75 mol%.
(12) The set of primers according to any one of (8) to (11), wherein, in the second primer, the 3' terminus of the second polynucleotide tag is linked to the 5' terminus of the second polynucleotide via an inhibitor that inhibits the nucleic acid amplification reaction.

(13) A kit for detecting a target nucleic acid comprising the set of primers according to any one of (1) to (7) and a nucleic acid detecting device comprising a solid-phase support, wherein the solid-phase support comprises a capture substance holding section comprising a capture substance capable of binding to the first polynucleotide tag.
(14) A kit for detecting a target nucleic acid comprising the set of primers according to any one of (8) to (12) and a nucleic acid detecting device comprising a solid-phase support, wherein the solid-phase support comprises a capture substance holding section comprising a capture substance capable of binding to the first polynucleotide tag and a label substance holding section comprising a label substance capable of binding to the second polynucleotide tag.
(15) The kit according to (13) or (14), wherein the nucleic acid detecting device further comprises a sample acceptor section that accepts a sample containing a nucleic acid amplified product.

(16) A method for detecting a target nucleic acid comprising:
   a step of nucleic acid amplification comprising subjecting a test material that may contain a target nucleic acid to a nucleic acid amplification reaction using the set of primers according to any one of (1) to (12); and
   a step of detection comprising detecting a target nucleic acid in a reaction product obtained in the step of nucleic acid amplification.
(17) The method according to (16), wherein, in the step of detection, the target nucleic acid is detected using a solid-phase support.
(18) The method according to (16) or (17), wherein, in the step of nucleic acid amplification, the nucleic acid amplification reaction is performed using a strand displacing polymerase.
(19) The method according to (18), wherein, in the step of nucleic acid amplification, the nucleic acid amplification reaction is performed by the RPA method, the TRIAmp method, the LAMP method, or the HDA method.
(20) The method according to (16) or (17), wherein, in the step of nucleic acid amplification, the nucleic acid amplification reaction is performed by PCR.
(21) A multiplex set of primers comprising two or more sets of primers according to any one of (1) to (12), wherein target nucleic acids amplified by the nucleic acid amplification reaction using the two or more sets of primers are different from each other.

This description encompasses the content disclosed in Japanese Patent Application No. 2019-239045, on which the priority of the present application is based.

### Effects of the Invention

The nucleic acid amplification reaction using the set of primers according to one or more embodiments of the present invention enables efficient preparation of an amplified product of a target nucleic acid with a polynucleotide tag that can be detected on a solid-phase support.

### Brief Description of the Drawings

### [Fig. 1-1]

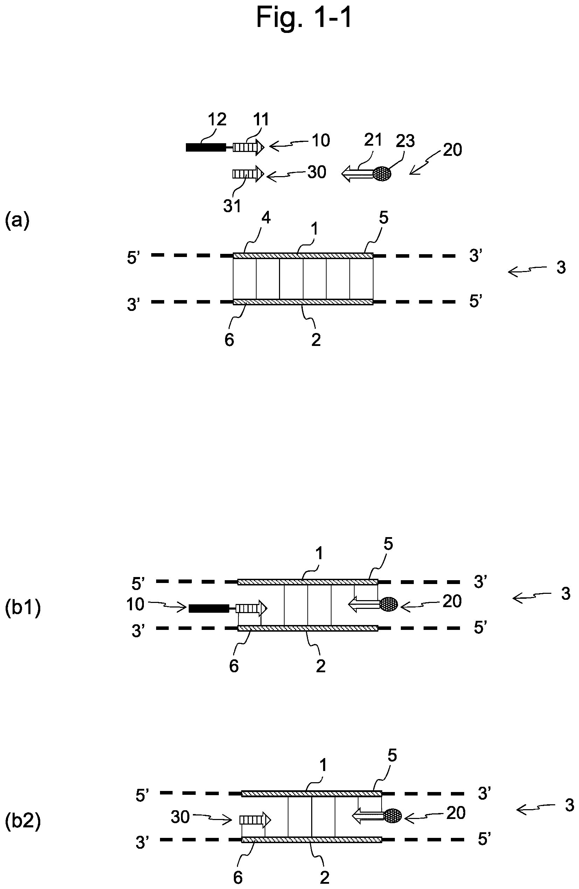

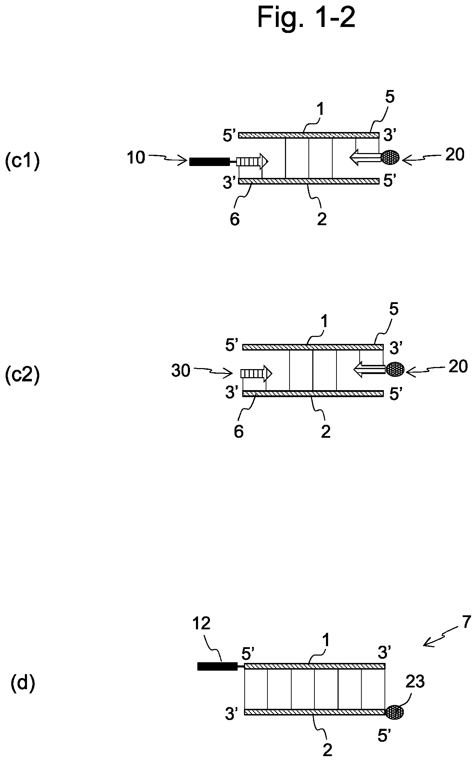

Fig. 1-1 (a) schematically shows an example of a test nucleic acid 3 comprising a target nucleic acid 1 and a complementary strand 2 of the target nucleic acid 1, the first primer 10, the second primer 20, and the third primer 30. Fig. 1-1 (b1) schematically shows an isothermal nucleic acid amplification reaction using the test nucleic acid 3 as a template, the first primer 10, and the second primer 20. Fig. 1-1 (b2) schematically shows an isothermal nucleic acid amplification reaction using the test nucleic acid 3 as a template, the third primer 30, and the second primer 20.

### [Fig. 1-2]

Fig. 1-2 (c1) shows a reaction in which the first primer 10 and the second primer 20 hybridize to a double strand of the target nucleic acid 1 and the complementary strand 2, which is an amplified product of the prior nucleic acid amplification reaction, and a polymerase extension reaction is initiated. Fig. 1-2 (c2) schematically shows an isothermal nucleic acid amplification reaction using the double strand of the target nucleic acid 1 and the complementary strand 2 as a template, the third primer 30, and the second primer 20. Fig. 1-2 (d) shows a nucleic acid for detection 7, which is a double-stranded nucleic acid of the target nucleic acid 1 with the first polynucleotide tag 12 linked to the 5' terminus and the complementary strand 2 with the label substance 23 linked to the 5' terminus.

### [Fig 2]

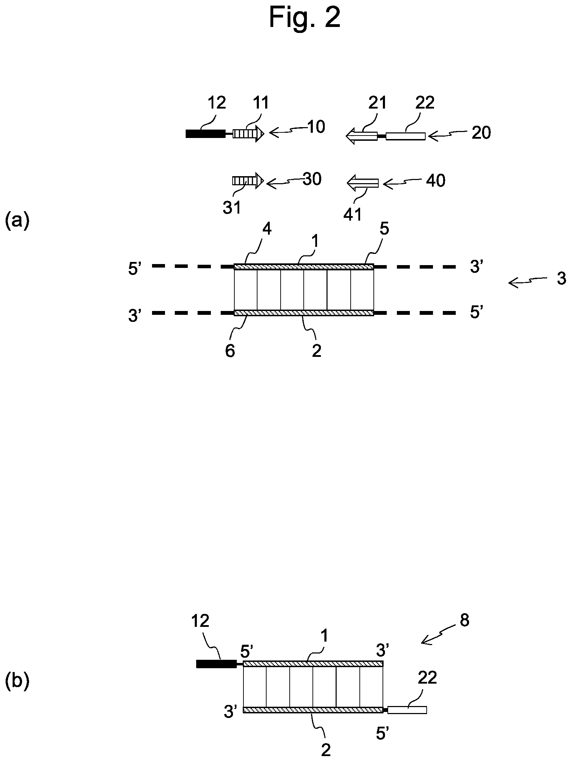

Fig. 2 (a) schematically shows an example of a test nucleic acid 3 comprising a target nucleic acid 1 and a complementary strand 2 of the target nucleic acid 1, the first primer 10, the second primer 20, the third primer 30, and the fourth primer 40. Fig. 2 (b) shows a nucleic acid for detection 8, which is a double-stranded nucleic acid of the target nucleic acid 1 with the first polynucleotide tag 12 linked to the 5' terminus and the complementary strand 2 with the second polynucleotide tag 22 linked to the 5' terminus.

### [Fig. 3]

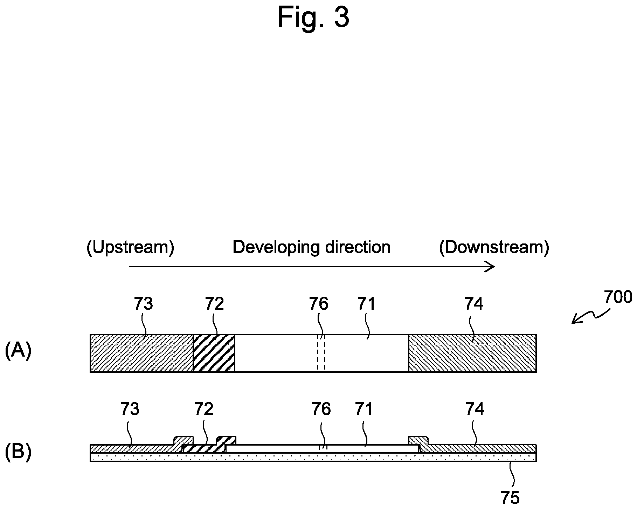

Fig. 3 schematically shows a lateral flow-type nucleic acid detecting device 700: 71: solid-phase support; 72: conjugate pad (label substance holding section); 73: sample pad (sample acceptor section); 74: absorbent pad; 75: substrate; 76: capture substance holding section.

### [Fig. 4]

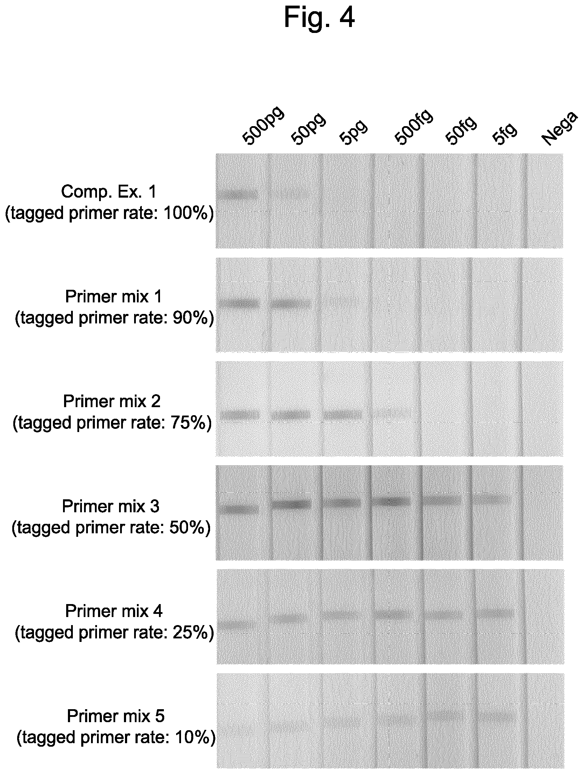

Fig. 4 shows the results of nucleic acid amplification of serially-diluted target nucleic acids at different concentrations by the TRIAmp method in Example 2 using, as forward primers and reverse primers, primers with DNA tags and primers without tags at various ratios.

### [Fig. 5]

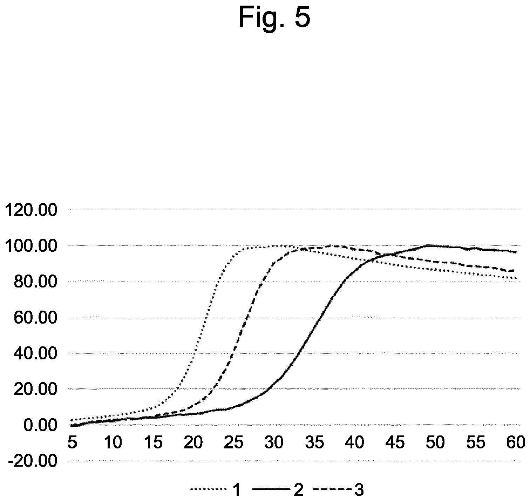

Fig. 5 shows the results of monitoring the nucleic acid amplification reaction of a target nucleic acid by the TRIAmp method using a real-time PCR apparatus in Example 4, using: a primer mix 1 of a forward primer and a reverse primer without DNA tags; a primer mix 2 of a forward primer with a DNA tag and a biotin-labeled reverse primer; or a primer mix 3 of a forward primer with a DNA tag, a forward primer without a DNA tag, and a biotin-labeled reverse primer.

### Embodiments of the Invention

Hereafter, one or more embodiments of the present invention are described in detail.

### <Terms>

The terms "nucleic acid" and "polynucleotide" used in one or more embodiments of the present invention refer to DNA or RNA, and the terms typically refer to DNA. The terms "nucleic acid" and "polynucleotide" also refer to an oligonucleotide without particular limitation in terms of the number of nucleotides. In one or more embodiments of the present invention, a target nucleic acid serving as a template in a nucleic acid amplification reaction, a complementary strand of the target nucleic acid, and a polynucleotide are typically a polymer of natural nucleotides. A natural nucleotide is composed of a base such as adenine, thymine, guanine, cytosine, or uracil, a sugar portion such as deoxyribose or ribose, and a phosphoric acid group, and none of such components are artificially modified. In general, a natural nucleotide is a D-nucleotide. The term "D-nucleotide" refers to a nucleotide comprising a sugar portion composed of D-form of deoxyribose or ribose.

A polynucleotide may be a partial polynucleotide comprised in a part of a relatively long polynucleotide. The phrase "polynucleotide P2 comprising polynucleotide PI" or "polynucleotide P2 comprises polynucleotide PI" encompass both of the case in which polynucleotide P1 constitutes the whole of polynucleotide P2 (i.e., polynucleotide P1 is identical to polynucleotide P2) and the case in which polynucleotide P1 is a partial polynucleotide of polynucleotide P2.

In one or more embodiments of the present invention, the term "target nucleic acid" refers to a nucleic acid comprising a nucleotide sequence to be detected and/or amplified or a nucleic acid comprising a nucleotide sequence complementary to the nucleotide sequence to be detected and/or amplified. A target nucleic acid may exsist as a double strand with its complementary strand. One strand of the target nucleic acid existing as a double strand may be referred to as a "target nucleic acid." Either of the nucleic acid to be detected or its complementary strand may be referred to as a "target nucleic acid." In one or more embodiments of the present invention, specifically, "detection of a target nucleic acid" and "amplification of a target nucleic acid" indicate both of the following situations. In one situation, a target nucleic acid is detected or amplified in order to detect or amplify the target nucleic acid itself. In the other situation, a target nucleic acid, a complementary strand of the target nucleic acid, or a double-stranded nucleic acid formed of the target nucleic acid and its complementary strand is detected or amplified in order to detect or amplify a complementary strand of the target nucleic acid or a double-stranded nucleic acid formed of the target nucleic acid and its complementary strand.

The full length of the target nucleic acid in one or more embodiments of the present invention is not particularly limited. In general, the target nucleic acid is 20 or more, 40 or more, or 100 or more nucleotide in length. The upper limit of the target nucleic acid full length is not particularly limited. In general, the upper limit is 1,000, 500, or 400 nucleotides.

A nucleic acid serving as a template in a nucleic acid amplification reaction in one or more embodiments of the present invention may be DNA or RNA, preferably DNA, provided that a part thereof comprises the target nucleic acid and/or its complementary strand. In general, a nucleic acid serving as a template in a nucleic acid amplification reaction is a double-stranded DNA composed of a polynucleotide strand comprising the target nucleic acid in at least a part thereof and a complementary strand of the polynucleotide strand.

A nucleic acid serving as a template may be a naturally-occurring or artificially synthesized nucleic acid. For example, it may be a naturally-occurring nucleic acid extracted from a biological sample, a nucleic acid amplified via PCR, or cDNA synthesized via reverse transcription.

In one or more embodiments of the present invention, the phrase: polynucleotide X "hybridizes" to polynucleotide Y means thatpolynucleotide X (DNA, in particular) hybridizes to polynucleotide Y (DNA, in particular) under stringent conditions but does not hybridize to a polynucleotide without the nucleotide sequence of polynucleotide Y. Namely, the word "hybridize" refers to specifically hybridizing. The term "stringent conditions" means conditions under which a so-called specific hybrid is formed, but a non-specific hybrid is not formed. Stringent conditions can be adequately determined with reference to, for example, Green and Sambrook, Molecular Cloning, 4th Ed., 2012, Cold Spring Harbor Laboratory Press. Specifically, stringent conditions can be determined by temperature or salt concentration in a solution during Southern hybridization and temperature or salt concentration in a solution during a washing step of Southern hybridization. More specifically, under stringent conditions, for example, the step of hybridization is performed at sodium concentration of 25 to 500 mM, and preferably 25 to 300 mM, and at 40°C to 68°C, and preferably at 40°C to 65°C. Further specifically, hybridization can be performed in 1 to 7× SSC (saline-sodium citrate buffer) 0.02% to 3% SDS at 40°C to 60°C. Hybridization may be followed by a washing step, and the washing step can be performed in, for example, 0.1 to 2× SSC, 0.1% to 0.3% SDS at 50°C to 65°C. It should be noted that hybridization between the first polynucleotide tag (a tag for fixing) described below and a solid-phase support with a polynucleotide, and hybridization between the second polynucleotide tag (a tag for labelling) described below and a label substance with a polynucleotide, are not necessarily performed under the stringent conditions described herein, and such hybridizations can be performed under the conditions described below.

When polynucleotide X hybridizes to polynucleotide Y, a combination of polynucleotide X (DNA, in particular) and polynucleotide Y (DNA, in particular) can form a sufficient hydrogen bond to form a stable double strand by hybridization under an annealing condition in the nucleic acid amplification reaction, and they are not necessarily formed of nucleotide sequences completely complementary to each other. For example, several mismatches, such as up to 1 mismatch in 10 nucleotides, up to 1 mismatch in 20 nucleotides, or up to 1 mismatch in 30 nucleotides may be present between the nucleotide sequence of polynucleotide X (hereafter, referred to as "nucleotide sequence X") and the nucleotide sequence of polynucleotide Y (hereafter, referred to as "nucleotide sequence Y").

When polynucleotide X hybridizes to polynucleotide Y, in general, one or more of the following conditions (A) to (C) are satisfied.

(A) A complementary nucleotide sequence of nucleotide sequence X is identical to nucleotide sequence Y. When one of the complementary nucleotide sequence of nucleotide sequence X and nucleotide sequence Y is a nucleotide sequence of DNA and the other is a nucleotide sequence of RNA, thymine in one sequence and uracil in the other sequence are considered the identical nucleotides.
(B) Nucleotide sequence Y is derived from a complementary sequence of nucleotide sequence X by deletion, substitution, addition, and/or insertion of one or several nucleotides.
(C) Nucleotide sequence Y has 80% or higher identity to the complementary sequence of nucleotide sequence X.

In (B) above, the term "one or several" refers to preferably 1 to 5, more preferably 1 to 4, more preferably 1 to 3, particularly preferably 1 or 2, and the most preferably 1.

In (C) above, sequence identity is calculated using software that computes identity among a plurality of nucleotide sequences (e.g., FASTA, DANASYS, and BLAST) at default settings. Nucleotide sequence identity is determined by aligning a pair of nucleotide sequences so as to maximize an extent of identity, determining the number of identical nucleotides therebetween, and determining a percentage of the number of identical nucleotides based on the total number of nucleotides in the compared nucleotide sequences. When there are gaps, the total number of nucleotides is determined by counting one gap as one nucleotide. A method for determining sequence identity is described in detail in, for example, Altschul et al., Nuc. Acids Res., 25, 3389-3402, 1977 and Altschul et al., J. Mol. Biol., 215, 403-410, 1990.

In (C) above, sequence identity is more preferably 90% or higher, more preferably 95% or higher, more preferably 96% or higher, more preferably 97% or higher, more preferably 98% or higher, and more preferably 99% or higher.

Among (A) to (C) above, (A) is particularly preferable.

The expression "polynucleotide X hybridizes to polynucleotide Y" is used in the same sense as the expression "nucleotide sequence X hybridizes to nucleotide sequence Y."

A method for producing polynucleotides constituting a set of primers according to one or more embodiments of the present invention is not particularly limited. Polynucleotides may be produced using a polynucleotide synthesizer or using synthesis services.

In the present description, a polynucleotide "independent of the nucleic acid amplification reaction" can also be expressed as a polynucleotide "not involved in a nucleic acid amplification reaction." A polynucleotide "independent of the nucleic acid amplification reaction" is not used as a template in the nucleic acid amplification reaction, and such polynucleotide does not form a double strand as a result of the nucleic acid amplification reaction.

### <Nucleic acid amplification reaction>

A nucleic acid amplification reaction using the set of primers according to one or more embodiments of the present invention may be a nucleic acid amplification reaction using a heat stable polymerase or a nucleic acid amplification reaction using a strand displacing polymerase. A polymerase refers to a nucleic acid polymerase, and it is a DNA polymerase or RNA polymerase, preferably a DNA polymerase.

An example of a nucleic acid amplification reaction using a heat stable polymerase is a polymerase chain reaction (PCR). A commercially available DNA polymerase can be used as a heat stable polymerase, and, for example, TaKaRa Ex Taq^{®} can be preferably used. Temperature, a duration, a buffer composition, and other conditions can be adequately determined in accordance with a DNA polymerase used, primer concentration, and the like. PCR conditions, such as a duration of each of denaturation, annealing, and extension steps, temperature, a buffer composition, substrate nucleotide concentration, and the number of cycles, can be adequately determined in view of elements, such as the DNA polymerase selected, primer sequences, the number of nucleotides constituting the target nucleic acid, and template concentration.

A strand displacing polymerase is an enzyme that dissociates a hydrogen bond of a double-stranded nucleic acid comprising a target nucleic acid and synthesizes a novel DNA strand. Examples thereof include ϕ29 DNA polymerase, Bst DNA polymerase, DNA polymerase I Klenow fragment, Vent DNA polymerase, Vent (Exo-) DNA polymerase, DeepVent DNA polymerase, DeepVent (Exo-) DNA polymerase, 96-7 DNA polymerase, Aac DNA polymerase, and Csa DNA polymerase. Since a strand displacing polymerase does not require thermal denaturation for dissociation of double strands, nucleic acid amplification can be performed under isothermal conditions.

A nucleic acid amplification reaction using a strand displacing polymerase is preferably an isothermal nucleic acid amplification method, and examples thereof include the RPA (recombinase polymerase amplification) method, the TRIAmp (tandem repeat-mediated isothermal amplification) method, the LAMP (loop-mediated isothermal amplification) method, and the HAD (helicase-dependent amplification) method.

In the RPA method and the TRIAmp method, a set of at least one primer pair is used to amplify a target nucleic acid. As a primerof the set of one primer pair, the first primer and the third primer of the set of primers according to one or more embodiments of the present invention can be used. As another primer, the second primer of the set of primers according to one or more embodiments of the present invention or the second primer and the fourth primer can be used.

In the LAMP method, 4 or more types of primers are used to amplify a target nucleic acid. As one primer of the 4 or more types of primers, the first primer and the third primer of the set of primers according to one or more embodiments of the present invention can be used in the LAMP method. As another primer, the second primer of the set of primers according to one or more embodiments of the present invention or a pair of the second primer and the fourth primer can be used. It is preferable that either or both of the one primer and another primer is a loop primer.

The isothermal nucleic acid amplification method using a strand displacing polymerase proceeds by incubating a template nucleic acid comprising a target nucleic acid, primers, a strand displacing polymerase, substrate nucleotides, and, as necessary, other enzymes (e.g., recombinase and a single-stranded DNA binding protein (SSB) in the RPA method) at a temperature at which the amplification primers can form stable base pair bonds with the template nucleic acid and anenzyme activity can be exerted. Conditions for the nucleic acid amplification reaction by the isothermal nucleic acid amplification method, such as temperature, a buffer composition, substrate nucleotide concentration, and the reaction duration, can be adequately determined in accordance with elements, such as strand displacing polymerases, primer sequences, the number of nucleotides constituting the target nucleic acid, and template nucleic acid concentration. Examples of recombinases used in the RPA method include UvsX, RecA, and analogs thereof.

### <Set of primers>

When a test material to be analyzed contains a nucleic acid comprising the nucleotide sequence of the target nucleic acid, a set of primers comprising the first primer, the second primer, and the third primer according to one or more embodiments of the present invention can prepare a nucleic acid for detection comprising a target nucleic acid to which the first polynucleotide tag is linked at one terminus by a nucleic acid amplification reaction using a target nucleic acid in the test material as a template.

Hereafter, primer structures are described and preferable embodiments of a set of primers are then described.

### (First primer)

The first primer comprises: the first polynucleotide comprising, at the 3' terminus, polynucleotide A capable of hybridizing to a complementary strand of partial polynucleotide A' at the 5' terminus of the target nucleic acid; and the first polynucleotide tag, which is a polynucleotide independent of the nucleic acid amplification reaction, linked to the 5' terminus of the first polynucleotide.

The partial polynucleotide A' is a partial polynucleotide of a target nucleic acid consisting of more than one continuous nucleotides, comprising a nucleotide at the 5' terminus of the target nucleic acid. The number of nucleotides constituting the partial polynucleotide A' is not particularly limited. For example, the number of nucleotides constituting the partial polynucleotide A' can be 8 or more, 10 or more, 12 or more, 15 or more, 17 or more, or 20 or more, and can be 40 or less, 30 or less, 27 or less, or 25 or less.

The polynucleotide A may be a polynucleotide capable of hybridizing to a complementary strand of the partial polynucleotide A'. In preferable embodiments of the polynucleotide A, a nucleotide sequence of continuous one or more nucleotides, preferably 2 or more nucleotides, more preferably 3 or more nucleotides, further preferably 5 or more nucleotides at the 3' terminus of the polynucleotide A, most preferably, the whole nucleotide sequence of the polynucleotide A, is identical to the nucleotide sequence in a corresponding region of the partial polynucleotide A'. The number of nucleotides constituting the polynucleotide A is not particularly limited, and can be adequately determined in accordance with the number of nucleotides constituting the partial polynucleotide A'. For example, the number of nucleotides constituting the polynucleotide A can be 8 or more, 10 or more, 12 or more, 15 or more, 17 or more, or 20 or more, and can be 40 or less, 30 or less, 27 or less, or 25 or less.

The nucleotide sequence of the first polynucleotide comprises, at its 3' terminus, the nucleotide sequence of the polynucleotide A. The nucleotide sequence of the first polynucleotide may further comprise another nucleotide sequence added to the 5' terminal side of the nucleotide sequence of the polynucleotide A or may consist of the nucleotide sequence of the polynucleotide A. Namely, the polynucleotide A may be a partial polynucleotide comprising the 3' terminus of the first polynucleotide or the whole of the first polynucleotide. The number of nucleotides constituting the first polynucleotide can be adequately determined in accordance with the number of nucleotides constituting the polynucleotide A. For example, the number of nucleotides constituting the first polynucleotide can be 8 or more, 10 or more, 12 or more, 15 or more, 17 or more, or 20 or more and can be 40 or less, 30 or less, 27 or less, or 25 or less.

The first primer comprises the first polynucleotide and the first polynucleotide tag, which is a polynucleotide independent of the nucleic acid amplification reaction, linked to the 5' terminus of the first polynucleotide. The first polynucleotide tag may be referred to as the "first tag" or a "tag for fixing." A polynucleotide comprised in the first polynucleotide tag is not particularly limited, provided that it does not substantially affect the nucleic acid amplification reaction using the set of primers according to one or more embodiments of the present invention. For example, such polynucleotide has 5 to 50, and preferably 10 to 35 nucleotides. A preferable example thereof is the polynucleotide comprising the nucleotide sequence described in Anal. Biochem., 364, 2007, 78-85.

The first polynucleotide tag can bind to a capture substance contained in the solid-phase support described below. The nucleic acid for detection comprising the target nucleic acid with the first polynucleotide tag can be fixed to a capture substance holding section of the solid-phase support and detected.

The 5' terminus of the first polynucleotide is linked to the first tag in such a manner that the first tag would not form a double strand together with the first polynucleotide that functions as a primer as a result of extension in the nucleic acid amplification reaction. The 5' terminus of the first polynucleotide may be linked to the 3' terminus of the first tag, or the 5' terminus of the first polynucleotide may be linked to the 5' terminus of the first tag. It is preferable that the 5' terminus of the first polynucleotide is linked to the 3' terminus of the first tag.

In one or more embodiments, the 5' terminus of the first polynucleotide is linked to the first tag via an inhibitor that inhibits the nucleic acid amplification reaction (hereafter, it may be referred to as a "spacer").

In other embodiments, the first tag can be a polynucleotide that would not serve as a template in a polymerase reaction and would not form a double strand as a result of extension in the nucleic acid amplification reaction, e.g., a polynucleotide comprising a modified nucleic acid, such as LNA (locked nucleic acid), L-nucleic acid, and 2'-O-methylated nucleotide. In such a case, the first polynucleotide may be directly linked to the first tag without the spacer.

The spacer is not particularly limited, provided that it can inhibit or terminate proceeding of the polymerase reaction in the nucleic acid amplification reaction and prevent the first tag from forminga double strand. Examples thereof include, but are not limited to, a nucleic acid sequence comprising a strong firm hairpin structure or pseudoknot structure, L-nucleic acid, peptide nucleic acid (PNA), bridged nucleic acid (BNA) or locked nucleic acid (LNA), fluorescein, Cy3, Cy5, a divalent group having an azobenzene structure represented by Formula I below, an aliphatic chain (an alkylene chain or a polyoxyalkylene chain), and a divalent group comprising an inverted sequence structure such as a 5'-5' bond or a 3'-3' bond. Using such spacer, the first polynucleotide and the first tag can be linked to each other in the same direction. Specifically, the 3' terminus of the first tag can be linkedto the 5' terminus of the first polynucleotide via the spacer described above.

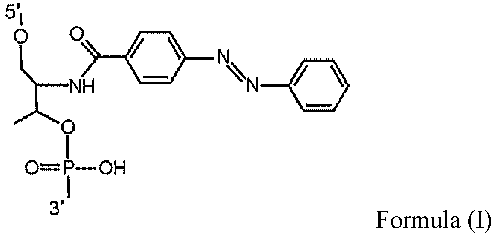

When two polynucleotide molecules are connected to each other via a divalent group represented by Formula I, a phosphoric acid group at the 3' terminus of the divalent group is a phosphoric acid group of a nucleotide at the 5' terminus of a polynucleotide molecule, and an oxygen atom at the 5' terminus forms a phosphoric acid ester bond with a phosphoric acid group of a nucleotide at the 3' terminus of the other polynucleotide molecule.

An example of an aliphatic chain spacer is represented by Formula (IV) below:

5'-O-CH₂ₘ-O-3' Formula (IV)

wherein
5' represents an oxygen atom of the 5'-terminal phosphodiester bond, 3' represents an oxygen atom of the 3'-terminal phosphodiester bond, m is an integer of 1 to 40, and H may be substituted with a substituent.

In Formula (IV), m is preferably 2 to 36, and more preferably 3 to 16. H in Formula (IV) may be substituted with a substituent, and typical examples of substituents include an alkyl group, an alkoxy group, and a hydroxyl group. An alkyl group and an alkoxy group as substituents preferably have 1 to 8, and more preferably 1 to 4 carbon atoms. When there are two or more substituents, such substituents may be the same with or different from each other. It is also preferable that there are no substituents.

An example of another spacer is represented by Formula (V) below:

5'-(OCₙH₂ₙ)_{L}-O-3' Formula (V)

wherein
5' represents an oxygen atom of the 5'-terminal phosphodiester bond, 3' represents an oxygen atom of the 3'-terminal phosphodiester bond, n is an integer of 2 to 4, L is an integer of 1 or greater that satisfies (n + 1) × L of 40 or smaller, and H may be substituted with a substituent.

In Formula (V), a value represented by (n + 1) × L is preferably 2 to 36, and more preferably 3 to 16. The substituent for H in Formula (V) is the same as the substituent in Formula (IV).

When two polynucleotide molecules form a 5'-5' bond, an aliphatic chain spacer represented by Formula (IV) or (V) above can be used.

Examples of other aliphatic chain spacers include the divalent groups demonstrated below.

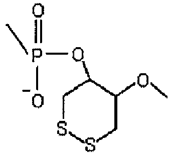

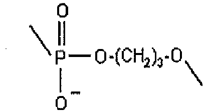

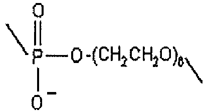

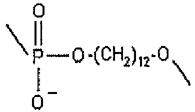

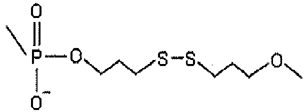

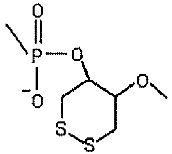

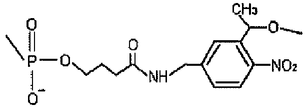

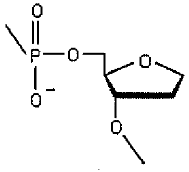

When two polynucleotide molecules are connected to each other via a divalent group demonstrated above, a phosphoric acid group at one terminus of the divalent group is a phosphoric acid group of a nucleotide at the 3' terminus or the 5' terminus of one polynucleotide molecule, and an oxygen atom at the other terminus forms a phosphoric acid ester bond with a phosphoric acid group of a nucleotide at the 5' terminus or the 3' terminus of the other polynucleotide molecule.

### (Second primer)

The second primer comprises the second polynucleotide comprising, at the 3' terminus, polynucleotide B capable of hybridizing to partial polynucleotide B' at the 3' terminus of the target nucleic acid.

The partial polynucleotide B' is a partial polynucleotide of a target nucleic acid consisting of more than one continuous nucleotides, comprising a nucleotide at the 3' terminus of the target nucleic acid. The number of nucleotides constituting the partial polynucleotide B' is not particularly limited. For example, the number of nucleotides constituting the partial polynucleotide B' can be 8 or more, 10 or more, 12 or more, 15 or more, 17 or more, or 20 or more, and can be 40 or less, 30 or less, 27 or less, or 25 or less.

The polynucleotide B may be a polynucleotide capable of hybridizing to the partial polynucleotide B'. In preferable embodiments of the polynucleotide B, a nucleotide sequence of continuous one or more nucleotides, preferably 2 or more nucleotides, more preferably 3 or more nucleotides, further preferably 5 or more nucleotides at the 3' terminus of the polynucleotide B, most preferably, the whole nucleotide sequence of the polynucleotide B is identical to the nucleotide sequence in a corresponding region of a complementary strand of the partial polynucleotide B'. The number of nucleotides constituting the polynucleotide B is not particularly limited and it can be adequately determined in accordance with the number of nucleotides constituting the partial polynucleotide B'. For example, the number of nucleotides constituting the polynucleotide B can be 8 or more, 10 or more, 12 or more, 15 or more, 17 or more, or 20 or more, and can be 40 or less, 30 or less, 27 or less, or 25 or less.

The nucleotide sequence of the second polynucleotide comprises, at its 3' terminus, the nucleotide sequence of the polynucleotide B. The nucleotide sequence of the second polynucleotide may further comprise another nucleotide sequence added to the 5' terminal side of the nucleotide sequence of the polynucleotide B or may consist of the nucleotide sequence of the polynucleotide B. Namely, the polynucleotide B may be a partial polynucleotide comprising the 3' terminus of the second polynucleotide or the whole of the second polynucleotide. The number of nucleotides constituting the second polynucleotide can be adequately determined in accordance with the number of nucleotides constituting the polynucleotide B. For example, the number of nucleotides constituting the second polynucleotide can be 8 or more, 10 or more, 12 or more, 15 or more, 17 or more, or 20 or more and can be 40 or less, 30 or less, 27 or less, or 25 or less.

In one or more embodiments of the present invention, the second primer can comprise the second polynucleotide and a label substance linked to the second polynucleotide. A nucleic acid for detection, which is an amplified product of the target nucleic acid by the set of primers comprising the second primer according to the embodiment, comprises the first polynucleotide tag at its one terminus and a label substance at the other terminus. The nucleic acid for detection can be captured on a solid-phase support having a capture substance holding section comprising a capture substance capable of binding to the first polynucleotide tag, and can be detected using a label substance as a marker.

A label substance is not particularly limited, provided that it does not inhibit a nucleic acid amplification reaction. A label substance itself can be a detectable label substance, or it may be a label substance capable of binding to another detectable substance via a secondary reaction.

Examples of label substances that do not inhibit the nucleic acid amplification reaction include biotin, dye, hapten, and a radioisotope-containing substance. Examples of dyes include fluorescent dyes (e.g., fluorescein, such as fluorescein isothiocyanate (FITC), and cyanine). Examples of haptens include digoxigenin (DIG) and fluorescein isothiocyanate (FITC).

Biotin can be detected via a secondary reaction with a detectable substance linked to avidin or streptavidin. Hapten can be detected via a secondary reaction with a detectable substance linked to an antibody. The detectable substance includes a dye, a radioactive substance, a colored particle, and an enzyme (e.g., peroxidase, alkaline phosphatase, and luciferase), and preferably a colored particle. Examples of the "colored particle" that can be used include a metal (e.g., gold, silver, copper, and platinum) particle, a metal rod, a colored latex particle, and a silica nanoparticle containing a dye.

A label substance can bind to the second polynucleotide at any position, provided that a nucleic acid amplification reaction is not inhibited, and, in general, a label substance binds to the 5' terminus of the second polynucleotide.

In one or more other embodiments of the present invention, the second primer comprises the second polynucleotide and the second polynucleotide tag, which is a polynucleotide independent of the nucleic acid amplification reaction, linked to the 5' terminus of the second polynucleotide. A nucleic acid for detection, which is an amplified product of the target nucleic acid by the set of primers comprising the second primer according to the embodiment, comprises the first polynucleotide tag at its one terminus and the second polynucleotide tag at the other terminus. The nucleic acid for detection can be captured on a solid-phase support having a capture substance holding section comprising a capture substance capable of binding to the first polynucleotide tag, allowed to bind to a label substance capable of binding to the second polynucleotide tag, and detected using the label substance as a marker. Preferably, the label substance holding section of the solid-phase support is impregnated with a label substance capable of binding to the second polynucleotide tag in advance, and when a sample containing the nucleic acid for detection applied to the solid-phase support is brought into contact with the label substance holding section, the second polynucleotide tag of the nucleic acid for detection is bound to the label substance.

The second polynucleotide tag may be referred to as the "second tag" or a "tag for labelling." A polynucleotide comprised in the second polynucleotide tag is not particularly limited, provided that it does not substantially affect the nucleic acid amplification reaction using the set of primers according to one or more embodiments of the present invention. For example, such polynucleotide has 5 to 50, and preferably 10 to 35 nucleotides. A preferable example thereof is the polynucleotide comprising the nucleotide sequence described in Anal. Biochem., 364, 2007, 78-85.

The 5' terminus of the second polynucleotide is linked to the second tag in such a manner that the second tag would not form a double strand together with the second polynucleotide that functions as a primer as a result of extension in the nucleic acid amplification reaction. The 5' terminus of the second polynucleotide may be linked to the 3' terminus of the second tag, or the 5' terminus of the second polynucleotide may be linked to the 5' terminus of the second tag. It is preferable that the 5' terminus of the second polynucleotide is linked to the 3' terminus of the second tag.

In one or more embodiments, the 5' terminus of the second polynucleotide is linked to the second tag via an inhibitor that inhibits the nucleic acid amplification reaction (i.e., a spacer). A spacer that connects the second tag to the 5' terminus of the second polynucleotide can be selected from among the spacers described as those connecting the first tag to the 5' terminus of the first polynucleotide.

In other embodiments, the second tag can be a polynucleotide that would not serve as a template in a polymerase reaction and would not form a double strand as a result of extension in the nucleic acid amplification reaction, e.g., a polynucleotide comprising a modified nucleic acid, such as LNA (locked nucleic acid), L-nucleic acid, and 2'-O-methylated nucleotide. In such a case, the second polynucleotide may be directly linked to the second tag without the spacer.

Preferably, a label substance capable of binding to the second tag is a label substance linked to a polynucleotide capable of hybridizing to the second tag (e.g., a polynucleotide comprising a sequence complementary to the nucleotide sequence of the second tag). Conditions for hybridization between the nucleic acid for detection comprising the second tag and a label substance linked to a polynucleotide capable of hybridizing to the second tag are not particularly limited, provided that hybridization can be carried out. For example, hybridization can be performed at 20°C to 40°C in a 10 mM to 50 mM phosphoric acid buffer (pH 6 to 7). A buffer solution can be supplemented with salt such as sodium chloride, so as to enhance hybridization efficiency.

The label substance linked to a polynucleotide capable of hybridizing to the second tag includes a colored particle and an enzyme (e.g., peroxidase, alkaline phosphatase, and luciferase) in addition to the above-mentioned label substances linked to the second polynucleotide, and preferably is a colored particle. Examples of the "colored particle" include a metal (e.g., gold, silver, copper, and platinum) particle, a metal rod, a colored latex particle, and a silica nanoparticle containing a dye.

### (Third primer)

The third primer comprises a third polynucleotide comprising, at the 3' terminus, polynucleotide C capable of hybridizing to the complementary strand of the target nucleic acid competitively with the polynucleotide A of the first primer, and comprises no polynucleotide independent of the nucleic acid amplification reaction.

The third primer preferably consists of the third polynucleotide.

The term "polynucleotide C capable of hybridizing to the complementary strand of the target nucleic acid competitively with the polynucleotide A of the first primer" refers to the correlation as follows. That is, when one of polynucleotide A of the first primer and polynucleotide C of the third primer hybridizes to the complementary strand of the target nucleic acid, the other cannot hybridize to the complementary strand of the same target nucleic acid. For example, when polynucleotide C of the third primer is capable of hybridizing to a polynucleotide having 50% or more, 60% or more, 80% or more, 90% or more, 95% or more, or 100% nucleotides within the complementary strand of the partial polynucleotide A' of the target nucleic acid (i.e., a region capable of hybridizing to the polynucleotide A of the first primer), the polynucleotide C is capable of hybridizing to the complementary strand of the target nucleic acid competitively with the polynucleotide A of the first primer.

In preferable embodiments of the polynucleotide C, a nucleotide sequence of continuous one or more nucleotides, preferably 2 or more nucleotides, more preferably 3 or more nucleotides, further preferably 5 or more nucleotides at the 3' terminus of the polynucleotide C, most preferably, the whole nucleotide sequence of polynucleotide C, is identical to the nucleotide sequence in a corresponding region of the partial polynucleotide A'. The number of nucleotides constituting the polynucleotide C is not particularly limited and can be adequately determined in accordance with the number of nucleotides constituting the partial polynucleotide A'. For example, the number of nucleotides constituting the polynucleotide C can be 8 or more, 10 or more, 12 or more, 15 or more, 17 or more, or 20 or more and can be 40 or less, 30 or less, 27 or less, or 25 or less. It is particularly preferable that the polynucleotide C is identical to the polynucleotide A.

The nucleotide sequence of the third polynucleotide comprises, at its 3' terminus, the nucleotide sequence of the polynucleotide C. The nucleotide sequence of the third polynucleotide may further comprise another nucleotide sequence added to the 5' terminal side of the nucleotide sequence of the polynucleotide C or may consist of the nucleotide sequence of the polynucleotide C. Namely, the polynucleotide C may be a partial polynucleotide comprising the 3' terminus of the third polynucleotide or the whole of the third polynucleotide. The number of nucleotides constituting the third polynucleotide can be adequately determined in accordance with the number of nucleotides constituting the polynucleotide C. For example, the number of nucleotides constituting the third polynucleotide can be 8 or more, 10 or more, 12 or more, 15 or more, 17 or more, or 20 or more and can be 40 or less, 30 or less, 27 or less, or 25 or less. It is particularly preferable that the third polynucleotide is identical to the first polynucleotide.

### (Mechanism of a nucleic acid amplification reaction using the set of primers)

Significance of using the third primer in combination with the first primer as forward primers of the target nucleic acid in an exemplary reaction in which the target nucleic acid is amplified by the isothermal nucleic acid amplification method using a set of primers according to one or more embodiments of the present invention, is described with reference to Fig. 1-1 and Fig. 1-2.

Fig. 1-1 (a) schematically shows an example of a test nucleic acid 3 comprising a target nucleic acid 1 and a complementary strand 2 of the target nucleic acid 1, the first primer 10, the second primer 20, and the third primer 30.

The test nucleic acid 3 is a double-stranded DNA comprising, in a part thereof, the target nucleic acid 1 and the complementary strand 2 hybridized to each other. The target nucleic acid 1 comprises the partial polynucleotide A' 4 at the 5' terminus and the partial polynucleotide B' 5 at the 3' terminus. The complementary strand 2 of the target nucleic acid 1 comprises the complementary strand 6 of the partial polynucleotide A' 4 at the 3' terminus.

The first primer 10 comprises: the first polynucleotide 11 comprising, at the 3' terminus, the polynucleotide A capable of hybridizing to the complementary strand 6 of the partial polynucleotide A' 4; and the first polynucleotide tag 12 independent of the nucleic acid amplification reaction linked to the 5' terminus of the first polynucleotide 11.

The second primer 20 comprises: the second polynucleotide 21 comprising, at the 3' terminus, the polynucleotide B capable of hybridizing to the partial polynucleotide B' 5; and the label substance 23.

The third primer 30 comprises the third polynucleotide 31 comprising, at the 3' terminus, the polynucleotide C capable of hybridizing to the complementary strand 2 of the target nucleic acid 1 competitively with the polynucleotide A within the first polynucleotide 11 of the first primer 10, and comprises no polynucleotide independent of the nucleic acid amplification reaction.

When amplifying the target nucleic acid 1 in the test nucleic acid 3 by the isothermal nucleic acid amplification method using the set of primers and a strand displacing polymerase, early-stage reactions; i.e., a reaction in which the first primer 10 and the second primer 20 hybridize to the test nucleic acid 3 to initiate a polymerase extension reaction shown in Fig. 1-1 (b1) and a reaction in which the third primer 30 and the second primer 20 hybridize to the test nucleic acid 3 to initiate a polymerase extension reaction shown in Fig. 1-1 (b2), are deduced to occur.

The first primer 10 comprises the first polynucleotide tag 12 that is not involved in the nucleic acid amplification reaction. Therefore, when hybridizing to the complementary strand 2 of the target nucleic acid 1 in the test nucleic acid 3 in the manner as shown in Fig. 1-1 (b1), accordingly, the first primer 10 is considered to have large steric hindrance. In addition, the nucleic acid amplification reaction using the strand displacing polymerase does not comprise a step of dissociating hydrogen bonds of the double-stranded test nucleic acid 3 at high temperature. Accordingly, the influence of the steric hindrance is considered to be more significant than that in PCR. In contrast, the third primer 30 does not comprise a polynucleotide that is not involved in the nucleic acid amplification reaction. Accordingly, the third primer 30 is considered to be more likely to hybridize to the complementary strand 2 of the target nucleic acid 1 in the test nucleic acid 3 in the manner as shown in Fig. 1-1 (b2). This indicates that the amplification reaction of the target nucleic acid 1 by the third primer 30 and the second primer 20 as shown in Fig. 1-1 (b2) is more likely to proceed but the amplification reaction involving the first primer 10 as shown in Fig. 1-1 (b1) is less likely to proceed, at the early stage of the nucleic acid amplification reaction.

When the nucleic acid amplification reaction proceeds, as shown in Fig. 1-2 (c1) and Fig. 1-2 (c2), an amplification reaction using a fragment of the target nucleic acid 1, which is an amplified product of the prior nucleic acid amplification reaction, and the complementary strand 2 of the target nucleic acid 1, as templates, becomes dominant. The influence of steric hindrance of the first primer 10 is insignificant on a double strand formed of the fragment of the target nucleic acid 1 and the complementary strand 2 of the target nucleic acid 1, which is the amplified product. Thus, both the reaction using the first primer 10 as a forward primer shown in Fig. 1-2 (c1) and the reaction using the third primer 30 shown in Fig. 1-2 (c2) proceed. In addition, an amplification reaction using, as a template, the fragment of the target nucleic acid 1 linked to the first polynucleotide tag 12 at the 5' terminus also proceeds (this reaction is not shown in Figures).

The final amplified product comprises the nucleic acid for detection 7 as shown in Fig. 1-2 (d), which is a double-stranded nucleic acid of the target nucleic acid 1 linked to the first polynucleotide tag 12 at the 5' terminus and the complementary strand 2 linked to the label substance 23 at the 5' terminus. The nucleic acid for detection 7 can be fixed to a solid-phase support having a capture substance holding section comprising a capture substance to bind to the first polynucleotide tag 12, and detected using the label substance 23 as a marker. The amplified product comprises a double-stranded nucleic acid formed of the target nucleic acid 1 without the first polynucleotide tag 12 at the 5' terminus and the complementary strand 2 linked to the label substance 20 at the 5' terminus (this amplified product is not shown in Figures).

Through the experiments described herein, the present inventors found that: reaction efficiency is low when the target nucleic acid 1 is amplified by PCR and isothermal nucleic acid amplification using only the first primer 10 as a forward primer (without using the third primer 30 in combination) and using the second primer 10 as a reverse primer, whereas reaction efficiency is improved to a significant extent when the first primer 10 is used in combination with the third primer 30 as forward primers. Such tendency was particularly apparent in the isothermal nucleic acid amplification method using the strand displacing polymerase than in PCR. The results of experiments demonstrate that an amplification reaction using the first primer 10 having a large steric hindrance is difficult to proceed at an early stage of the nucleic acid amplification reaction and the efficiency of the amplification reaction is low when only the first primer 10 is used as a forward primer. According to the embodiment of the present invention using the first primer 10 in combination with the third primer 30, in contrast, the amplification reaction of the target nucleic acid 1 by the third primer 30 and the second primer 20 primarily proceeds at an early stage of the nucleic acid amplification reaction, and the amplification reaction of the target nucleic acid 1 by the first primer 10 and the second primer 20 also proceeds as generation of the target nucleic acid 1 proceeds. Thus, the nucleic acid for detection 7 detectable on the solid-phase support can be efficiently obtained.

### (Amount ratio of the first primer and the third primer)

In the set of primers according to one or more embodiments of the present invention, the amount ratio of the first primer and the third primer is not particularly limited and can be adequately adjusted. For example, the proportion of the third primer relative to the total amount of the first primer and the third primer can be preferably 1 mol% or more, more preferably 2.5 mol% or more, more preferably 10 mol% or more, more preferably 25 mol% or more, and the most preferably 40 mol% or more, and can be preferably 90 mol% or less, and more preferably 75 mol% or less.

### (Embodiment of a set of primers further comprising a fourth primer)

As described above, the second primer may further comprise, in addition to the second polynucleotide, the second polynucleotide tag, which is polynucleotide independent of the nucleic acid amplification reaction, linked to the 5' terminus of the second polynucleotide. When only the second primer comprising the second polynucleotide tag is used as a reverse primer, efficiency for the early-stage nucleic acid amplification reaction tends to lower because of the steric structure of the second primer comprising the second polynucleotide tag, as with the case in which only the first primer is used as a forward primer.

Therefore, when the second primer comprising the second polynucleotide tag is used as a reverse primer, it is preferable that a fourth primer is used in combination therewith, and such fourth primer comprises a fourth polynucleotide comprising, at the 3' terminus, polynucleotide D capable of hybridizing to the target nucleic acid competitively with the polynucleotide B of the second primer, and comprises no polynucleotide independent of the nucleic acid amplification reaction.

The fourth primer preferably consists of the fourth polynucleotide.

The term "polynucleotide D capable of hybridizing to the target nucleic acid competitively with the polynucleotide B of the second primer" refers to the correlation as follows. That is, when one of polynucleotide B of the second primer and polynucleotide D of the fourth primer hybridizes to the target nucleic acid, the other cannot hybridize to the same target nucleic acid. For example, when the polynucleotide D of the fourth primer is capable of hybridizing to a polynucleotide having 50% or more, 60% or more, 80% or more, 90% or more, 95% or more, or 100% nucleotides within the partial polynucleotide B' of the target nucleic acid (i.e., a region capable of hybridizing to the polynucleotide B of the second primer), the polynucleotide D is capable of hybridizing to the target nucleic acid competitively with the polynucleotide B of the second primer.

In preferable embodiments of the polynucleotide D, a nucleotide sequence of continuous one or more nucleotides, preferably 2 or more nucleotides, more preferably 3 or more nucleotides, further preferably 5 or more nucleotides at the 3' terminus of the polynucleotide D, most preferably, the whole nucleotide sequence of the polynucleotide D is identical to the nucleotide sequence in a corresponding region of the complementary strand of the partial polynucleotide B'. The number of nucleotides constituting the polynucleotide D is not particularly limited and can be adequately determined in accordance with the number of nucleotides constituting the partial polynucleotide B'. For example, the number of nucleotides constituting the polynucleotide D can be 8 or more, 10 or more, 12 or more, 15 or more, 17 or more, or 20 or more, and can be 40 or less, 30 or less, 27 or less, or 25 or less. It is particularly preferable that the polynucleotide D be identical to the polynucleotide B.

The nucleotide sequence of the fourth polynucleotide comprises, at its 3' terminus, the nucleotide sequence of the polynucleotide D. The nucleotide sequence of the fourth polynucleotide may further comprise another nucleotide sequence added to the 5' terminal side of the nucleotide sequence of the polynucleotide D or may consist of the nucleotide sequence of the polynucleotide D. Namely, the polynucleotide D may be a partial polynucleotide comprising the 3' terminus of the fourth polynucleotide or the whole of the fourth polynucleotide. The number of nucleotides constituting the fourth polynucleotide can be adequately determined in accordance with the number of nucleotides constituting the polynucleotide D. For example, the number of nucleotides constituting the fourth polynucleotide can be 8 or more, 10 or more, 12 or more, 15 or more, 17 or more, or 20 or more, and can be 40 or less, 30 or less, 27 or less, or 25 or less. It is particularly preferable that the fourth polynucleotide be identical to the second polynucleotide.

In the present embodiment, the amount ratio of the second primer and the fourth primer is not particularly limited and can be adequately adjusted. For example, the proportion of the fourth primer relative to the total amount of the second primer and the fourth primer can be preferably 1 mol% or more, more preferably 2.5 mol% or more, more preferably 10 mol% or more, more preferably 25 mol% or more, and the most preferably 40 mol% or more, and can be preferably 90 mol% or less, and more preferably 75 mol% or less.

Fig. 2 (a) schematically shows an example of a test nucleic acid 3 comprising a target nucleic acid 1 and a complementary strand 2 of the target nucleic acid 1, the first primer 10, the second primer 20, the third primer 30, and the fourth primer 40. Features of the target nucleic acid 1, the complementary strand 2, the test nucleic acid 3, the first primer 10, and the third primer 30 in the example shown in Fig. 2 are the same as those shown in Fig. 1-1 and Fig. 1-2, and description thereof is thus omitted here.

In the present embodiment, the second primer 20 comprises: the second polynucleotide 21 comprising, at the 3' terminus, polynucleotide B capable of hybridizing to the partial polynucleotide B' 5; and the second polynucleotide tag 22 independent of the nucleic acid amplification reaction linked to the 5' terminus of the second polynucleotide 21.

The fourth primer 40 comprises a fourth polynucleotide 41 comprising, at the 3' terminus, polynucleotide D capable of hybridizing to the target nucleic acid 1 competitively with the polynucleotide B within the second polynucleotide 21 of the second primer 20, and comprises no polynucleotide independent of the nucleic acid amplification reaction.

The final amplified product prepared by the nucleic acid amplification reaction using the first primer 10, the second primer 20, the third primer 30, and the fourth primer 40 according to the embodiment shown in Fig. 2 (a) and, using the test nucleic acid 3 as a template, comprises a nucleic acid for detection 8, which is a double-stranded nucleic acid of the target nucleic acid 1 linked to the first polynucleotide tag 12 atthe 5' terminus and the complementary strand 2 linked to the second polynucleotide tag 22 at the 5' terminus, shown in Fig. 2 (b). The nucleic acid for detection 8 can be captured on a solid-phase support having a capture substance holding section comprising a capture substance capable of binding to the first polynucleotide tag 12, allowed to bind to a label substance capable of binding to the second polynucleotide tag 22, and detected using the label substance as a marker.

### <Detection of an amplified product of the target nucleic acid>

One or more embodiments of the present invention also relate to a method for detecting a target nucleic acid comprising:
a step of nucleic acid amplification comprising subjecting a test material that may contain a target nucleic acid to a nucleic acid amplification reaction using the set of primers according to one or more embodiments of the present invention; and
a step of detection comprising detecting the target nucleic acid in a reaction product obtained in the step of nucleic acid amplification.

In one or more embodiments of the present invention, an analyte sample containing the test material is not particularly limited, and typical examples include food and beverage products and samples containing parts of organisms such as animals and plants (e.g., organs, tissue, cells, blood, and body fluid), feces, microorganisms, and viruses.

A test material obtained from the analyte sample can be used in the nucleic acid amplification reaction. The test material may be a nucleic acid extracted from the analyte sample and purified, or a nucleic acid extracted from the analyte sample and roughly purified. When the test material is a nucleic acid, it may be referred to as a "test nucleic acid." When a sample contains the test nucleic acid at relatively high concentration, such as parts of cells or tissue, the analyte sample itself can be directly subjected to the nucleic acid amplification reaction. Also, cDNA prepared from the analyte sample can be used as a template. The test material used as a template is preferably DNA.

Hereafter, preferable embodiments of the step of nucleic acid amplification, the step of detection, and the step of labeling performed as necessary are described.

### (1. The step of nucleic acid amplification)

Preferable embodiments of the nucleic acid amplification reaction in the step of nucleic acid amplification are as described above.

When the test material contains the target nucleic acid, a nucleic acid for detection containing the target nucleic acid linked to the first polynucleotide tag at its one terminus, can be obtained by using the set of primers according to one or more embodiments of the present invention in the step of nucleic acid amplification.

When two or more different target nucleic acids are to be detected, the nucleic acid amplification reaction can be performed on the test material obtained from the analyte sample using a multiplex set of primers that is a combination of two or more sets of the primers mentioned above designed to amplify two or more target nucleic acids. The multiplex set of primers is described below.

### (2. Step of detection)

The step of detection comprises detecting a target nucleic acid in a reaction product obtained in the step of nucleic acid amplification. In the step of detection, preferably, a reaction product is brought into contact with a solid-phase support having a capture substance holding section comprising a capture substance capable of binding to the first polynucleotide tag, and the nucleic acid for detection is detected in the capture substance holding section of the solid-phase support.

The term "a reaction product obtained in the step of nucleic acid amplification" refers to, for example, a reaction solution obtained in the nucleic acid amplification reaction that may contain the nucleic acid for detection as an amplified product or a sample prepared by concentrating the amplified product obtained from the reaction solution.

In an embodiment in which the set of primers used in the step of nucleic acid amplification comprises the second primer comprising a label substance linked thereto, the nucleic acid for detection comprises a label substance ligated thereto. Thus, the nucleic acid for detection can be detected using the label substance as a marker.

In an embodiment in which the set of primers used in the step of nucleic acid amplification comprises the second primer comprising the second polynucleotide tag linked thereto, the nucleic acid for detection comprises the second polynucleotide tag linked thereto. Thus, a step of labeling in which the label substance is bound to the second polynucleotide tag can further be performed.

A solid-phase support is not particularly limited, and a solid-phase support made of, for example, resin, metal, polysaccharide, or mineral can be used. A solid-phase support can be in the form of, for example, a membrane, film, unwoven fabric, plate, or gel. A preferable solid-phase support has a porous structure, so that an amplified product or a label substance in a solution can spread. Examples of solid-phase supports that can be used include a filter paper, a nitrocellulose membrane, a polyether sulfone membrane, a nylon membrane, various types of dehydrated gel (e.g., silica gel, agarose gel, dextran gel, and gelatin gel), silicon, glass, and plastic. A size and a shape of a solid-phase support suitable for various procedures and detection can be adequately selected.

A capture substance held in the capture substance holding section of the solid-phase support is not particularly limited, provided that it can bind to the first polynucleotide tag. A preferable capture substance is a polynucleotide capable of hybridizing to the first polynucleotide tag. A particularly preferable polynucleotide capable of hybridizing to the first polynucleotide tag is a polynucleotide comprising a complementary nucleotide sequence of the first polynucleotide tag. The nucleotide sequence of the first polynucleotide tag and the nucleotide sequence of the polynucleotide used as a capture substance can be easily modified. Thus, binding specificity can be easily controlled. For example, when a sample containing a plurality of nucleic acids for detection containing different target nucleic acids are spread and detected on a solid-phase support, the first primers may be designed to add a first polynucleotide tag with a different nucleotide sequence to each of the plurality of nucleic acids for detection, and capture substance holding sections holding a polynucleotide capable of specifically hybridizing to the first polynucleotide tag of each of the nucleic acids for detection may be provided at a plurality of different positions on the solid-phase support.

A capture substance may be held in the capture substance holding section of the solid-phase support via a chemical bond, or it may be held therein by impregnating the capture substance holding section of the porous solid-phase support with the capture substance.

The reaction product may be brought into contact with the capture substance holding section of the solid-phase support under adequately modified conditions, so that the first polynucleotide tag of the nucleic acid for detection can bind to the capture substance holding section when the reaction product contains the nucleic acid for detection (e.g., the conditions under which the specific hybridization described above is formed or in a buffer with a pH of approximately 5 to 9).

The nucleic acid for detection containing the target nucleic acid can be detected in the manner described below. When the nucleic acid for detection captured in the capture substance holding section of the solid-phase support contains a label substance, the nucleic acid for detection can be detected by detecting, preferably detecting via visual inspection, the label substance.

### (3. Step of labeling)

The step of labeling is performed when the second primer of the set of primers contains the second polynucleotide tag or when the second primer of the set of primers contains a label substance capable of binding to another substance detectable via a secondary reaction. In such a case, the second polynucleotide tag or the label substance is added in the nucleic acid for detection containing the target nucleic acid. In the step of labeling, the reaction product is brought into contact with a label substance or a detectable substance, and thereby binding the label substance or the detectable substance to the second polynucleotide tag in the nucleic acid for detection containing the target nucleic acid. The step of labeling may be performed before, after, or at the same time that the reaction product is brought into contact with the solid-phase support.

Specific examples of label substances capable of binding to the second polynucleotide tag are as described with respect to the second primer.

### <Kit according to one or more embodiments of the present invention>

One or more embodiments of the present invention also relate to a kit for detecting a target nucleic acid comprising:
the set of primers according to one or more embodiments of the present invention and a nucleic acid detecting device comprising a solid-phase support, wherein
the solid-phase support comprises a capture substance holding section comprising a capture substance capable of binding to the first polynucleotide tag.

In an embodiment in which the set of primers comprises the second primer comprising the second polynucleotide tag linked thereto, it is preferable that the solid-phase support further comprise, in addition to the capture substance holding section, a label substance holding section containing a label substance capable of binding to the second polynucleotide tag.

The kit according to one or more embodiments of the present invention can further comprise a PCR buffer, dNTPs, DNA polymerase, a developing solution for nucleic acid chromatography, and the like.

Features of the solid-phase support are as described above.

Using the nucleic acid detecting device comprising the solid-phase support, the presence or absence of the nucleic acid for detection in the reaction product can be detected and determined, and the results can be attained in a simple and rapid manner.

Fig. 3 schematically shows an embodiment of a nucleic acid detecting device that can be used in one or more embodiments of the present invention, although the nucleic acid detecting device is not limited to this embodiment. In the following description, a numeral reference applied to each member corresponds to the numeral reference used in Fig. 3.

The nucleic acid detecting device 700 shown in Fig. 3 is formed by placing the following components on a substrate 75: a sample pad 73, which is a sample acceptor section to accommodate the reaction product obtained in the step of nucleic acid amplification; a conjugate pad 72, which is a label substance holding section to hold a label substance; a porous solid-phase support 71, which comprises, in a part thereof, a capture substance holding section 76 holding a capture substance capable of binding to the first polynucleotide comprised in the nucleic acid for detection; and an absorbent pad 74, so that these components are in contact with each other in the above order. The capture substance holding section 76 of the solid-phase support 71 is a region where the capture substance is localized and fixed. The sample pad 73, the conjugate pad 72, the solid-phase support 71, and the absorbent pad 74 can be formed of members with porous structures that can be used for the solid-phase support. These components may be formed of the same member or different members. The substrate 75 is not particularly limited, provided that it can support various members thereon and facilitate operation of the nucleic acid detecting device 700. For example, a substrate made of paper, resin, metal, or mineral can be used. When mixing a label substance in a developing solution, or when detecting an amplified product obtained using the set of primers comprising the second primer with a label substance added in advance, the conjugate pad 72 can be omitted. The nucleic acid detecting device 700 may be partially covered with a film of polyester or the like in order to prevent contamination or prevent liquid evaporation from the solid-phase support surface during the test.

A sample containing the reaction product of the nucleic acid amplification reaction obtained in the step of nucleic acid amplification is added to the sample pad 73. The reaction product of the nucleic acid amplification reaction may be added directly as the sample, or the reaction product may be added together with an adequate developing solution (e.g., a phosphate buffer, a Tris buffer, a Goods' buffer, or an SSC buffer). A developing solution can further be supplemented with a surfactant, salt, protein, nucleic acid, or the like, as neccessary. The sample added to the sample pad 73 develops downward in the direction indicated by the arrow in Fig. 3 by the capillary action.

In another embodiment, the sample pad 73 of the nucleic acid detecting device 700 can be soaked in the sample to be detected and/or the developing solution accommodated in a container (e.g., a PCR tube, an Eppendorf tube, or a 96-well plate) to develop the sample.

In the nucleic acid detecting device to be soaked in a container containing a sample containing the reaction product of the nucleic acid amplification reaction and/or the developing solution, the sample pad 73 can be omitted from the nucleic acid detecting device 700 shown in Fig. 3. In addition, the end of the solid-phase support 71 on the substrate 75 which is not adjacent to the absorbent pad 74 may be directly soaked in the sample and/or the developing solution.

When detecting an amplified product obtained by the set of primers comprising the second primer with the second polynucleotide tag added thereto, the nucleic acid for detection is bound to and labeled with a label substance when it passes through the conjugate pad 72, which is a label substance holding section holding the label substance capable of binding to the second polynucleotide tag.

When the nucleic acid for detection passes through the solid-phase support 71, subsequently, the nucleic acid for detection is brought into contact with the capture substance of the capture substance holding section 76, and it is then captured by and bound to the capture substance holding section 76.

When the reaction product contains the nucleic acid for detection, the label substance bound to the nucleic acid for detection captured by and bound to the capture substance holding section 76 of the solid-phase support 71 is detected. In the case that the lavel substance is visually observable, the capture substance holding section 76 gives a color due to the label substance. The presence or absence of the nucleic acid for detection can be determined based on whether the label substance is detected or not (i.e., presence or absence of coloration), and the presence or absence of the target nucleic acid in the test material can be determined based on the results of the detection.

### <Multiplex set of primers>

One or more other embodiments of the present invention relate to a multiplex set of primers comprising two or more sets of primers according to one or more embodiments of the present invention described above, wherein target nucleic acids amplified by the nucleic acid amplification reaction using the two or more sets of primers are different from each other.

In these embodiments, preferably, the first polynucleotide tags of the first primers comprised in the two or more sets of primers havenucleotide sequences different from each other, and each of the first polynucleotide tags binds to a different capture substance.

A solid-phase support used to detect a reaction product of the nucleic acid amplification reaction using the multiplex set of primers, preferably, comprises two or more capture substance holding sections holding capture substances different from each other at different positions on the solid-phase support. In such embodiments, preferably, each capture substance is a polynucleotide capable of hybridizing to one of first polynucleotide tags of the first primers comprised in two or more sets of primers.

### Examples

### <Example 1>

### (1) Primer design

Primers used in the TRIAmp reaction were prepared based on the forward primer DRa21 (SEQ ID NO: 1) and the reverse primer DRb19 (SEQ ID NO: 2) targeting the repeat sequence of tuberculosis described in Patent Document 4.

DRa21-Tag1 was prepared, by linking the 3' terminal side of the DNA tag sequence (Tag1)(SEQ ID NO: 3) for capturing it on a membrane in nucleic acid chromatography to the 5' terminal side of the forward primer DRa21 via a divalent group represented by Formula I above containing an azobenzene construct as a polymerase inhibitor.
Tag1: 5'-ATGCTACCGTATGCCCAGTG-3' (SEQ ID NO: 3)

DRb19-Tag2 was prepared by linking the 3' terminal side of the DNA tag sequence (Tag2) (SEQ ID NO: 4) for gold colloid labeling to the 5' terminal side of the reverse primer DRb19 via a divalent group represented by Formula I above containing an azobenzene construct as a polymerase inhibitor.
Tag2: 5'-GACAACGGAGACAGAGCCAA-3' (SEQ ID NO: 4)

Table 1 show the primers used.

**[Table 1]**

| Primer name | Sequence (5'-3') | SEQ ID NO: |
|---|---|---|
| DRa21 | CGGGGTTTTGGGTCTGACGAC | 1 |
| DRb19 | CCCGAGAGGGGACGGAAAC | 2 |
| DRa21-Tag1 | Tag1-CGGGGTTTTGGGTCTGACGAC | |
| DRb19-Tag2 | Tag2-CCCGAGAGGGGACGGAAAC | |

### (2) Preparation of oligonucleotide-biding gold colloids

Gold Colloid (40 nm, 9.0 × 10¹⁰ (the number of particles/ml), British Biocell International) was mixed with a thiol group-containing oligonucleotide represented by SEQ ID NO: 5 below, and incubated at 50°C for 16 hours. Then, this was centrifugated at 6,000 rpm for 15 minutes, and the supernatant was removed. 0.05 M sodium chloride and 5 mM phosphate buffer (pH 7) were added thereto and mixed therewith, and the resulting mixture was incubated again at 50°C for 40 hours.

After the incubation, the mixture was centrifugated at 6,000 rpm for 15 minutes, the supernatant was removed, and 5 mM phosphate buffer (pH 7) was added thereto. Such buffer replacement was performed again.

The prepared gold colloid solution was applied evenly on a grass fiber pad and dehydrated using a vacuum drier to prepare a conjugate pad. (Thiol group-containing oligonucleotide): 5'-TTGGCTCTGTCTCCGTTGTC-SH-3' (SEQ ID NO: 5)

Thiol group-containing oligonucleotide represented by SEQ ID NO: 5 is formed by binding of a phosphoric acid group at position 3' of cytosine nucleotide at the 3' terminus and a hydroxyl group of a compound represented by: HO-(CH₂)ₘ-SH (m is 6) via a phosphoric acid ester bond.

### (3) Preparation of a membrane holding a tag capture substance

A solution containing the oligonucleotide probe represented by SEQ ID NO: 6 below (a tag capture substance) was applied to a nitrocellulose membrane (Hi-Flow 180, Merck Millipore) in a line with a width of 1 mm orthogonal to the developing direction using a dispenser. Then, the membrane was dried at 40°C for 30 minutes to prepare a membrane with a tag capture substance holding section.
(Oligonucleotide probe): 5'-CACTGGGCATACGGTAGCAT-3' (SEQ ID NO: 6)

### (4) Preparation of a lateral flow-type nucleic acid detecting device

A lateral flow-type nucleic acid detecting device was prepared in accordance with the detecting device schematically shown in Fig. 3.

Namely, a polypropylene backing sheet (Lohmann) as the substrate 75; the conjugate pad prepared in (2) above as the conjugate pad 72; a membrane comprising the tag capture substance holding section prepared in (3) above as the solid-phase support 71 comprising the capture substance holding section 76; a glass fiber sample pad as the sample pad 73; and a cellulose absorbent pad as the absorbent pad 74, were adhered to overlap with each other as shown in Fig. 7 to prepare the lateral flow-type nucleic acid detecting device 700.

### (5) TRIAmp reaction

The primer mixes shown in Table 2 were prepared using the primers described in (1) above.

**[Table 2]**

| Primer name | Ex. 1 Primer mix | | | | |
|---|---|---|---|---|---|
| | 1 | 2 | 3 | 4 | 5 |
| DRa21 | **20 µM** | **18 µM** | **10 µM** | **2 µM** | **-** |
| DRb19 | **20 µM** | **18 µM** | **10 µM** | **2 µM** | **-** |
| DRa21-Tag1 | **-** | **2 µM** | **10 µM** | **18 µM** | **20 µM** |
| DRb19-Tag2 | **-** | **2 µM** | **10 µM** | **18 µM** | **20 µM** |

Subsequently, a 2x TRIAmp buffer mixture consisting of 40 mM Tris buffer (pH 8.8), 20 mM potassium chloride, 20 mM ammonium sulfate, 0.2% Tween 20, 1.8 M betaine, 1.6 mM deoxynucleotide triphosphate, and 12 mM magnesium sulfate was prepared.

Subsequently, 2 µl of the DNA sample was added to 12.5 µl of the TRIAmp buffer mixture, 2 µl each of the primer mix, 8 units of Bst DNA polymerase, and 5 µl of 10,000-fold diluted SYBR Green I solution to prepare 25 µl (the total amount) of the TRIAmp reaction solution. As the DNA sample, a DNA solution extracted from the *Mycobacterium bovis* BCG strain and purified (100 pg/µl) was used.

The reaction was performed using the real-time PCR apparatus (LightCycler 96, Roche) at 68°C for 1 hour, and fluorescence was measured over time.

After the reaction, 5 µl of the reaction solution was added to the sample pad of the lateral flow-type nucleic acid detecting device, 80 µl of the developing solution was added, and coloring lines were visually evaluated. "+++": very intense coloration was evaluated. "++": coloration was evaluated. "+": weak coloration was evaluated. "-": no coloration was evaluated.

The results are shown in Table 3.

**[Table 3]**

| | Time^{∗} | Visual evaluation |
|---|---|---|
| Primer mix 1 | 20 min | - |
| Primer mix 2 | 21 min | + |
| Primer mix 3 | 25 min | ++ |
| Primer mix 4 | 36 min | ++ |
| Primer mix 5 | 46 min | +++ |

| | | |
|---|---|---|
| ^{∗}Time at which fluorescence level reached 0.2 | | |

When the primer mix 5 (all primers of which were primers with DNA tags) was used, the TRIAmp reaction proceeded; however, the reaction speed was lowered to a significant extent, in comparison with the primer mix 1 (which comprises no primers with DNA tags). When the primer mixes 2 to 4 (in which some of the primers of the primer mix 5 were replaced with primers without DNA tags) were used, in contrast, the reaction speed was higher than the primer mix 5. When the primer mixes 3 and 2 (a half or more primers of which were replaced) were used, the time required to obtain a positive evaluation was shortened almost by half compared to the primer mix 5. Any primer mix comprising primers with DNA tags enabled detection based on coloring line of the lateral flow-type nucleic acid detecting device.

The above-described results demonstrate that the TRIAmp reaction would be inhibited when using primers with DNA tags, but the inhibited reaction would be improved to a significant extent by adding primers without DNA tags thereto.

### <Example 2>

It was demonstrated in Example 1 that the use of primers with DNA tags would lower the speed of the TRIAmp reaction. It was also demonstrated that replacement of some primers with DNA tags with primers without DNA tags would suppress lowering in the reaction speed. In Example 1, however, the amount of the amplified product was measured based on fluorescence of SYBR Green I. Therefore, it was not verified whether or not the amount of the amplified products with DNA tags that can be detected with the nucleic acid detecting device had actually increased.

Primers without DNA tags were mixed with primers with DNA tags at various percentages, and the lower limit of detection was tested using a serially-diluted solution of DNA extracted from the *Mycobacterium bovis* BCG strain. The primers used in Example 1 were used. An experiment without the addition of the DNA sample containing the target nucleic acid was performed as a negative control experiment (Nega). Table 4 shows the compositions of primer mixes. As a comparative example, the primer mix consisting of primers with DNA tags was prepared (Comparative Example 1).

**[Table 4]**

| Primer name | Ex. 2 Primer mix | | | | |
|---|---|---|---|---|---|
| | 1 | 2 | 3 | 4 | 5 |
| DRa21 | **2 µM** | **5 µM** | **10 µM** | **15 µM** | **18 µM** |
| DRb19 | **2 µM** | **5 µM** | **10 µM** | **15 µM** | **18 µM** |
| DRa21-Tag1 | **18 µM** | **15 µM** | **10 µM** | **5 µM** | **2 µM** |
| DRb19-Tag2 | **18 µM** | **15 µM** | **10 µM** | **5 µM** | **2 µM** |

The TRIAmp reaction was performed in the same manner as in Example 1, except that SYBR Green I was not added. After the reaction, 5 µl of the reaction solution was added to the sample pad of the lateral flow-type nucleic acid detecting device, 80 µl of the developing solution was added, and coloring lines were visually evaluated. "+++": very intense coloration was evaluated. "++": coloration was evaluated. "+": weak coloration was evaluated. "-": no coloration was evaluated.

The results are shown in Fig. 4 and Table 5. In the comparative example in which a primer mix consisting of primers with DNA tags was used, 50 pg or more could be detected. In contrast, the primer mix 1 comprising primers without DNA tags could detect 5 pg or more, the primer mix 2 could detect 500 fg or more, and the primer mixes 3 to 5 could detect 5 fg or more. For the primer mixes 4 and 5 with the tagged primer rate of 25% or less, the coloring lines tended to fade. The primer mix 3 with the tagged primer rate of 50% exhibited the most satisfactory performance in terms of the lower limit of detection and the coloring line density.

**[Table 5]**

| | 500 pg | 50 pg | 5 pg | 500 fg | 50 fg | 5 fg | Nega |
|---|---|---|---|---|---|---|---|
| Comp. Ex. 1 (tagged primer rate: 100%) | +++ | + | - | - | - | - | - |
| Primer mix 1 (tagged primer rate: 90%) | +++ | +++ | + | - | - | - | - |
| Primer mix 2 (tagged primer rate: 75%) | +++ | +++ | +++ | + | - | - | - |
| Primer mix 3 (tagged primer rate: 50%) | +++ | +++ | +++ | +++ | +++ | +++ | - |
| Primer mix 4 (tagged primer rate: 25%) | ++ | ++ | ++ | ++ | ++ | ++ | - |
| Primer mix 5 (tagged primer rate: 10%) | + | + | + | + | + | + | - |

### <Comparative Example 2>

A primer mix composed of the forward primer DRa21 (SEQ ID NO: 1) and the reverse primer DRb19 (SEQ ID NO: 2) without DNA tags was prepared. In the same manner as in Example 1, the reaction and fluorescence assay were performed using a real-time PCR apparatus. A serially-diluted solution of DNA extracted from the *Mycobacterium bovis* BCG strain was used as a DNA sample. An experiment without the addition of the DNA sample containing the target nucleic acid was performed as the negative control experiment (Nega).

The results are shown in Table 6. The lower limit of detection for the primers without DNA tags was 100 fg. The lower limit of detection for the primer mixes 3 to 5 in Example 2 was 5 fg. It was demonstrated that with the use of the primer mix of the constitution according to one or more embodiments of the present invention, detection can be performed with higher sensitivity without the use of a real-time PCR apparatus, in comparison with a conventional technique.

**[Table 6]**

| | | 1ng | 100 pg | 10 pg | 1 pg | 100 fg | 10 fg | Nega |
|---|---|---|---|---|---|---|---|---|
| Comp. Ex. 2 | Time^{∗}(min) | 18.75 | 23.17 | 27.07 | 33.97 | 41.59 | - | - |
| | Evaluation | Positive | Positive | Positive | Positive | Positive | Negative | Negative |

| | | | | | | | | |
|---|---|---|---|---|---|---|---|---|
| ^{∗}Time at which fluorescence level reached 0.2 | | | | | | | | |

### <Example 3>

Experiment was performed in the same manner as in Example 2, except for the use of the primer mixes shown in Table 7.

**[Table 7]**

| Primer name | Comp. Ex. 3 | Ex. 3 Primer mix | | |
|---|---|---|---|---|
| | | 1 | 2 | 3 |
| DRa21 | - | 0.2 µM | 0.5 µM | 1 µM |
| DRb19 | - | 0.2 µM | 0.5 µM | 1 µM |
| DRa21-Tag1 | 20 µM | 19.8 µM | 19.5 µM | 19 µM |
| DRb19-Tag2 | 20 µM | 19.8 µM | 19.5 µM | 19 µM |

The results are shown in Table 8. The primer mix 1 with the tagged primer rate of 99% (i.e., the percentage of primers with DNA tags based on the total amount of primers) showed the lower limit of detection equivalent to that of Comparative Example 3, and an improvement was observed in the line coloring intensity at the lower limit of detection of 5 p. With the use of primer mixes with the tagged primer rates of 97.5% and 95%, 500 fg of DNA was detected, and an improvement was observed in terms of the lower limit of detection.

**[Table 8]**

| | 500 pg | 50 pg | 5 pg | 500 fg | Nega |
|---|---|---|---|---|---|
| Comp. Ex. 3 | +++ | +++ | ++ | - | - |
| Primer mix 1 (tagged primer rate: 99%) | +++ | +++ | +++ | - | - |
| Primer mix 2 (tagged primer rate: 97.5%) | +++ | +++ | +++ | + | - |
| Primer mix 3 (tagged primer rate: 95%) | +++ | +++ | +++ | ++ | - |

### <Example 4>

The primer mixes shown in Table 9 were prepared using DRb19-biotin (in which the 5' terminus of the DRb19 primer was modified with biotin as a label substance in accordance with a conventional technique) instead of DRb19-Tag2. The conjugate pad of the lateral flow-type nucleic acid detecting device was prepared using streptavidin-binding gold colloids as detectable biotin-binding substances. Otherwise, experiment was performed in the same manner as in Example 1.

**[Table 9]**

| Primer name | Primer mix | | |
|---|---|---|---|
| | 1 | 2 | 3 |
| DRa21 | 20 µM | - | 10 µM |
| DRb19 | 20 µM | - | - |
| DRa21-Tag1 | - | 20 µM | 10 µM |
| DRb19-biotin | - | 20 µM | 20 µM |

Fig. 5 shows the results of fluorescence assay using a real-time PCR apparatus. In comparison with the primer mix 1 comprising forward primers without DNA tags, initiation of amplification was delayed to a significant extent for the primer mix 2 in which all the forward primers had been replaced with primers with DNA tags. The speed of amplification was improved to a significant extent for the primer mix 3 in which a half of forward primers had been replaced with primers without DNA tags.

### <Example 5>

Using the primer mixes 2 and 3 of Example 4, the lower limit of detection was tested in the same manner as in Example 3.

The results are shown in Table 10. While the lower limit of detection was 500 fg for the primer mix 2 with the tagged primer rate of 100%, the primer mix 3 with the tagged primer rate of 50% could detect 50 fg. This experiment showed sensitivity improvement effects of using the primer mix with the constitution according to one or more aspects of the present invention.

**[Table 10]**

| | 500 pg | 50 pg | 5 pg | 500 fg | 50 fg | Nega |
|---|---|---|---|---|---|---|
| Ex. 4 Primer mix 2 | +++ | +++ | +++ | ++ | - | - |
| Ex. 4 Primer mix 3 | +++ | +++ | +++ | +++ | ++ | - |

### <Example 6>

Whether or not the sensitivity improvement effects would be attained using the primer mix of the constitution according to one or more embodiments of the present invention in the RPA method was examined.

### (1) Primer design

As primers used in the RPA reaction, the forward primer fts Y-1F (SEQ ID NO: 7) and the reverse primer fts Y-1R (SEQ ID NO: 8) specifically binding to the ftsY gene of *Staphylococcus aureus* were designed.

ftsY-1F-Tag3 was prepared by linking the 3' terminal side of the DNA tag sequence (Tag3) for capturing it on a membrane in nucleic acid chromatography to the 5' terminal side of the forward primer ftsY-1F via a divalent group represented by Formula I above containing an azobenzene construct as a polymerase inhibitor.
Tag3: 5'-TCGAGTGACAGCTAATGTGTGAT-3' (SEQ ID NO: 9)

fts Y-1R-Tag2 was prepared by linking the DNA tag sequence (Tag2) (SEQ ID NO: 4) for gold colloid labeling to the 5' terminal side of the reverse primer fts Y-1R via a divalent group represented by Formula I above containing an azobenzene construct as a polymerase inhibitor.

Table 11 shows the primers used in Example 6.

**[Table 11]**

| Primer name | Sequence (5'-3') | SEQ ID NO: |
|---|---|---|
| ftsY-1F | GAACAAGGTCAAGACAAATTAGA | 7 |
| ftsY-1R | TCCTGCGTTGAACCTTCAGA | 8 |
| ftsY-1F-Tag3 | Tag3-GAACAAGGTCAAGACAAATTAGA | |
| ftsY-1F-Tag2 | Tag2-CCCGAGAGGGGACGGAAAC | |

### (2) Preparation of a membrane holding a tag capture substance

A solution containing the oligonucleotide probe represented by SEQ ID NO: 10 below (a tag capture substance) was applied to a nitrocellulose membrane (Hi-Flow 180, Merck Millipore) in a line with a width of 1 mm orthogonal to the developing direction using a dispenser. Then, the membrane was dried at 40°C for 30 minutes to prepare a membrane with a tag capture substance holding section.
(Oligonucleotide probe): 5'-ATCACACATTAGCTGTCACTCGA-3' (SEQ ID NO: 10)

### (3) Preparation of a lateral flow-type nucleic acid detecting device

A lateral flow-type nucleic acid detecting device was prepared in the same manner as in Example 1, except that the membrane comprising the tag capture substance holding section prepared in (2) above was used as the solid-phase support 71.

### (4) RPA reaction

The RPA reaction was performed using the TwistAmp Basic kit (TwistDx). As a primer mix, a mixture of primers with DNA tags and primers without DNA tags at the proportion shown in Table 12 was used. As Comparative Example 4, a primer mix consisting of primers with DNA tags was prepared.

The primer mix (4.8 µl), 29.5 µl of Primer Free Rehydration Buffer, 2 µl of a serially-diluted solution of DNA extracted from *Staphylococcus aureus* (ATCC25923) (5 ng/µl to 50 fg/µl), and 11.2 µl of sterile distilled water were mixed to prepare a rehydration solution. The rehydration solution was added to a tube containing RPA freeze-dried reaction pellets to rehydrate the reagent. Then, 2.5 µl of 280 mM magnesium acetate was added and mixed, and the resulting mixture was subjected to the reaction at 37°C for 20 minutes.

**[Table 12]**

| Primer name | Comp. Ex. 4 | Ex. 6 Primer mix | | |
|---|---|---|---|---|
| | | 1 | 2 | 3 |
| ftsY-1F | **-** | **2.5 µM** | **5 µM** | **7.5 µM** |
| ftsY-1R | **-** | **2.5 µM** | **5 µM** | **7.5 µM** |
| ftsY-1F-Tag3 | **10 µM** | **7.5 µM** | **5 µM** | **2.5 µM** |
| ftsY-1F-Tag2 | **10 µM** | **7.5 µM** | **5 µM** | **2.5 µM** |

After the reaction, 5 µl of the reaction solution was added to the sample pad of the lateral flow-type nucleic acid detecting device, 80 µl of the developing solution was added, and coloring lines were visually evaluated. "+++": very intense coloration was evaluated. "++": coloration was evaluated. "+": weak coloration was evaluated. "-": no coloration was evaluated.

The results are shown in Table 13. In Comparative Example 4 in which a primer mix consisting of primers with DNA tags was used, the lower limit of detection was 10 ng. For the primer mixes 1 to 3, in contrast, the lower limit of detection was 1 pg to 10 pg. It was thus verified that the lower limit of detection would be improved in the presence of primers without DNA tags.

**[Table 13]**

| | lOng | 1ng | 100 pg | 10 pg | 1pg | 100 fg | Nega |
|---|---|---|---|---|---|---|---|
| Comp. Ex. 4 (tagged primer rate: 100%) | + | - | - | - | - | - | - |
| Primer mix 1 (tagged primer rate: 75%) | +++ | +++ | +++ | + | - | - | - |
| Primer mix 2 (tagged primer rate: 50%) | +++ | +++ | +++ | + | + | - | - |
| Primer mix 3 (tagged primer rate: 25%) | ++ | ++ | ++ | + | - | - | - |

### <Example 7>

Whether or not the effects of sensitivity improvement would be attained with the use of the primer mix of the constitution according to one or more embodiments of the present invention by the LAMP method was examined.

### (1) Primer design

As primers used in the LAMP reaction, the set of primers amplifying the Nuc gene of *Staphylococcus aureus* described in Non-Patent Document 1 (Frontiers in Microbiology, vol. 8, Article 192) was used. Table 14 shows the primers used.

**[Table 14]**

| Primer name | **Sequence (5'-3')** | SEQ ID NO: |
|---|---|---|
| Sau-F3 | ATGCAAAGAAAATTGAAGTCGA | 11 |
| Sau-B3 | GCGTTGTCTTCGCTCCAAAT | 12 |
| Sau-FIP | CGTTTACCATTTTTCCATCAGCATAGTTTGACAAAGGTCAAAGAACT | 13 |
| Sau-BIP | TCAAGGCTTGGCTAAAGTTGCTTATTTTCGCTTGTGCTTCACTT | 14 |
| Sau-LF | TACGCTAAGCCACGTCCATA | 15 |
| Sau-LB | CCTAACAATACACATGAACAAC | 16 |

Sau-LF-Tag1 was prepared by linking the 3' terminal side of the DNA tag sequence (Tag1) (SEQ ID NO: 3) for capturing it on a membrane in nucleic acid chromatography to the 5' terminal side of the loop primer Sau-LF (SEQ ID NO: 15) via a divalent group represented by Formula I above containing an azobenzene construct as a polymerase inhibitor.

Sau-Tag2 was prepared by linking the 3' terminal side of the DNA tag sequence (Tag2) (SEQ ID NO: 4) for gold colloid labeling to the 5' terminal side of the loop primer Sau-LB (SEQ ID NO: 16) via a divalent group represented by Formula I above containing an azobenzene construct as a polymerase inhibitor.

### (2) LAMP reaction

A reaction solution comprising 20 mM Tris buffer (pH 8.8), 10 mM potassium chloride, 10 mM ammonium sulfate, 0.1% Tween 20, 0.9 M betaine, 0.8 mM deoxynucleotide triphosphate, 6 mM magnesium sulfate, 8 units of Bst DNA polymerase, 1.6 µM Sau-FIP, 1.6 µM Sau-BIP, 0.8 µM Sau-F3, 0.8 µM Sau-B3, 0.4 µM Sau-LF, 0.4 µM Sau-LB, 0.4 µM Sau-LF-Tagl, and 0.4 µM Sau-LB-Tag2 was used. As a comparative example, a reaction solution using 0.8 µM Sau-LF and 0.8 µM Sau-LB as the loop primer of the above-described reaction solution was prepared. To the resulting reaction solution, 2 µl of a DNA sample was added, and the reaction was allowed to proceed at 62°C for 1 hour.

After the reaction, 5 µl of the reaction solution was added to the sample pad of the lateral flow-type nucleic acid detecting device as used in Example 2, and 80 µl of the developing solution was added. After dehydration, the line coloring intensity was assayed using an immunochromatoreader (Hamamatsu Photonics K.K.).

The line coloring intensity of the comparative example in which all the loop primers were primers with DNA tags was 25 mAbs. In contrast, when a half of the loop primers was replaced with primers without DNA tags, the line coloring intensity was 50 mAbs, and an increase in the amount of tagged products was observed.

### <Example 8>

Whether or not the sensitivity improvement effects would be attained using the primer mix of the constitution according to one or more embodiments of the present invention in PCR method was examined.

### (1) PCR

In order to examine PCR, the primer mixes shown in Table 15 were prepared using the same primers as used in Example 6. As Comparative Example 5, a primer mix consisting of primers with DNA tags was prepared.

**[Table 15]**

| Primer name | Comp. Ex. 5 | Ex. 8 Primer mix | | |
|---|---|---|---|---|
| | | 1 | 2 | 3 |
| ftsY-1F | **-** | **0.25 µM** | **0.5 µM** | **0.75 µM** |
| ftsY-1R | **-** | **0.25 µM** | **0.5 µM** | **0.75 µM** |
| ftsY-1F-Tag3 | **1 µM** | **0.75 µM** | **0.5 µM** | **0.25 µM** |
| ftsY-1F-Tag2 | **1 µM** | **0.75 µM** | **0.5 µM** | **0.25 µM** |

A reaction solution was prepared in accordance with the instruction of 2× HSTaq perfect Mix UNG plus (TakaraBio). The 1 µM primer mix (2 µl), 2 µl of a DNA sample extracted from *Staphylococcus aureus* (100 µg/µl to 10 fg/µl), and 6 µl of sterile distilled water were added to 10 µl of 2× HSTaq perfect Mix to prepare 20 µl of a PCR solution.

The PCR solution was mounted on a thermal cycler (LifeEco, Bior), the reaction was allowed to proceed at 94°C for 3 minutes, and a cycle of 94°C for 5 seconds, 58°C for 5 seconds, and 72°C for 15 seconds was repeated 35 times.

After the reaction, 5 µl of the reaction solution was added to the sample pad of the lateral flow-type nucleic acid detecting device, 80 µl of the developing solution was added, and coloring lines were visually examined. "+++": very intense coloration was evaluated. "++": coloration was evaluated. "+": weak coloration was evaluated. "-": no coloration was evaluated.

The results are shown in Table 16. In Comparative Example 5 in which a primer mix consisting of primers with DNA tags was used, the lower limit of detection was 20 ng. The primer mixes 1 to 3, in contrast, could detect 2 pg or more. It was thus verified that the lower limit of detection would be improved in the presence of primers without DNA tags.

**[Table 16]**

| | 200 pg | 20 pg | 2 pg | 200 fg | 20 fg | Nega |
|---|---|---|---|---|---|---|
| Comp. Ex. 5 (tagged primer rate: 100%) | ++ | + | - | - | - | - |
| Primer mix 1 (tagged primer rate: 75%) | +++ | ++ | + | - | - | - |
| Primer mix 2 (tagged primer rate: 50%) | +++ | +++ | + | - | - | - |
| Primer mix 3 (tagged primer rate: 25%) | ++ | ++ | + | - | - | - |

All publications, patents, and patent applications cited herein are incorporated herein

## Claims

1. A set of primers for preparing an amplified product of a target nucleic acid that can be detected on a solid-phase support via a nucleic acid amplification reaction,
wherein the set of primers comprises a first primer, a second primer, and a third primer,
wherein the first primer comprises:
a first polynucleotide comprising, at the 3' terminus, polynucleotide A capable of hybridizing to a complementary strand of partial polynucleotide A' at the 5' terminus of a target nucleic acid; and
a first polynucleotide tag, which is a polynucleotide independent of the nucleic acid amplification reaction, linked to the 5' terminus of the first polynucleotide,
wherein the second primer comprises a second polynucleotide comprising, at the 3' terminus, polynucleotide B capable of hybridizing to partial polynucleotide B' at the 3' terminus of the target nucleic acid, and
wherein the third primer comprises a third polynucleotide comprising, at the 3' terminus, polynucleotide C capable of hybridizing to the complementary strand of the target nucleic acid competitively with the polynucleotide A of the first primer, and comprises no polynucleotide independent of the nucleic acid amplification reaction.

2. The set of primers according to claim 1, wherein the polynucleotide C of the third primer is capable of hybridizing to a polynucleotide having 50% or more nucleotides within a complementary strand of the partial polynucleotide A' of the target nucleic acid.

3. The set of primers according to Claim 1 or 2, wherein the proportion of the third primer to the total amount of the first primer and the third primer is 1 mol% to 90 mol%.

4. The set of primers according to Claim 3, wherein the proportion of the third primer to the total amount of the first primer and the third primer is 2.5 mol% to 75 mol%.

5. The set of primers according to any one of Claims 1 to 4, wherein the second primer further comprises a label substance linked to the second polynucleotide.

6. The set of primers according to any one of Claims 1 to 4,
wherein the set of primers further comprises a fourth primer,
wherein the second primer further comprises a second polynucleotide tag, which is a polynucleotide independent of the nucleic acid amplification reaction, linked to the 5' terminus of the second polynucleotide, and
wherein the fourth primer comprises a fourth polynucleotide comprising, at the 3' terminus, polynucleotide D capable of hybridizing to the target nucleic acid competitively with the polynucleotide B of the second primer, and comprises no polynucleotide independent of the nucleic acid amplification reaction.

7. The set of primers according to Claim 6, wherein the polynucleotide D of the fourth primer is capable of hybridizing to a polynucleotide having 50% or more nucleotides within the partial polynucleotide B' of the target nucleic acid.

8. The set of primers according to Claim 6 or 7, wherein the proportion of the fourth primer to the total amount of the second primer and the fourth primer is 1 mol% to 90 mol%.

9. The set of primers according to Claim 8, wherein the proportion of the fourth primer to the total amount of the second primer and the fourth primer is 2.5 mol% to 75 mol%.

10. A kit for detecting a target nucleic acid comprising the set of primers according to any one of Claims 1 to 5 and a nucleic acid detecting device comprising a solid-phase support, wherein the solid-phase support comprises a capture substance holding section comprising a capture substance capable of binding to the first polynucleotide tag.

11. A kit for detecting a target nucleic acid comprising the set of primers according to any one of Claims 6 to 9 and a nucleic acid detecting device comprising a solid-phase support, wherein the solid-phase support comprises a capture substance holding section comprising a capture substance capable of binding to the first polynucleotide tag and a label substance holding section comprising a label substance capable of binding to the second polynucleotide tag.

12. A method for detecting a target nucleic acid comprising:
a step of nucleic acid amplification comprising subjecting a test material that may contain a target nucleic acid to a nucleic acid amplification reaction using the set of primers according to any one of Claims 1 to 9; and
a step of detection comprising detecting a target nucleic acid in a reaction product obtained in the step of nucleic acid amplification.

13. The method according to Claim 12, wherein, in the step of detection, the target nucleic acid is detected using a solid-phase support.

14. The method according to Claim 12 or 13, wherein, in the step of nucleic acid amplification, the nucleic acid amplification reaction is performed using a strand displacing polymerase.

15. A multiplex set of primers comprising two or more sets of primers according to any one of Claims 1 to 9, wherein target nucleic acids amplified by the nucleic acid amplification reaction using the two or more sets of primers are different from each other.
